# EUROPÄISCHE PATENTSCHRIFT

(11) **EP 1 373 175 B1**
(45) Veröffentlichungstag und Bekanntmachung des Hinweises auf die Patenterteilung: **08.07.2009**
(21) Anmeldenummer: 02745196.2
(22) Anmeldetag: 22.03.2002
(51) Int. Cl.: C07C 22/00, C09K 19/32, C07C 43/192

(54) **2,4'-SUBSTITUIERTE 6-CYCLOHEXYL-TRANS-DEKALINE**
2,4'-SUBSTITUTED 6-CYCLOHEXYL-TRANS-DECALINES
6-CYCLOHEXYL-TRANS-DECALINES SUBSTITUEES AUX POSITIONS 2,4'

(30) Priorität: 07.04.2001 DE 10117559
(43) Veröffentlichungstag der Anmeldung: 02.01.2004
(73) Patentinhaber: Merck Patent GmbH, 64293 Darmstadt (DE)
(72) Erfinder: BREMER, Matthias, 64295 Darmstadt (DE); PAULUTH, Detlef, 64372 Ober-Ramstadt (DE); LÜSSEM, Georg, 85238 Petershausen (DE)
(86) Internationale Anmeldenummer: PCT/EP2002/003228
(87) Internationale Veröffentlichungsnummer: WO 2002/081418

(56) Entgegenhaltungen:
- WO-A1-02/46329
- DE-A- 3 150 312
- DE-A- 10 112 952
- JP-A- 2000 355 557
- SUCROW W. ET AL.: "Flüssig-kristalline 2-Cyclohexyldecaline" CHEMISCHE BERICHTE., Bd. 118, Nr. 8, 1985, Seiten 3350-3356, XP002228536 VERLAG CHEMIE GMBH. WEINHEIM., DE ISSN: 0009-2940 in der Anmeldung erwähnt

## Beschreibung

Die Erfindung betrifft 2,4'-substituierte 6-Cyclohexyl-trans-Dekaline der allgemeinen Formel I 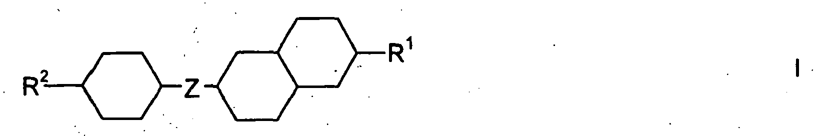 worin
- R¹: Halogen oder Alkyl mit 1 bis 18 C-Atomen, worin auch eine oder zwei nicht benachbarte CH₂-Gruppen durch -O-, -S-, -CO-, -O-CO-, -CO-O- und/ oder -C≡C- ersetzt sein können, und worin mindestens ein H-Atom durch ein Halogen-Atom substituiert ist, bedeutet,
- R²: H, Halogen, -CN oder Alkyl mit 1 bis 18 C-Atomen, worin auch eine oder zwei nicht benachbarte -CH₂-Gruppen durch -O-, -S-, -CO-, -O-CO-, -CO-O- und/ oder -C≡C- ersetzt sein können und/ oder worin auch eine oder mehrere H-Atome durch Halogen ersetzt sein können, bedeutet, und
- Z: Einfachbindung, -CH₂O-, -OCH₂-, -COO-, -C≡C-, -CH=CH-, -CF₂O-, -OCF₂-, -CF=CF-, -C₂F₄-, -CH=CH-(CH₂)₂- oder (CH₂)₄-,

Der Anmelder hat sich unter Bezugnahme auf DE 10 112 952 A freiwillig eingeschränkt und gesonderte Patentansprüche für DE vorgelegt.

Weiterhin betrifft die Erfindung die Verwendung solcher Verbindungen als Komponenten flüssigkristalliner Medien, ein solches flüssigkristallines Medium sowie ein Flüssigkristall- und ein elektrooptisches Anzeigeelement.

Aus der DE 31 50 312 A1 sind Dekaline der oben angegebenen allgemeinen Formel bekannt, in der R² Methyl oder eine der Gruppen -CH₂R', -OR', -CO-R', -CN, -COOH, -CO-OR', -CO-SR' oder -O-CO-R' bezeichnet; R¹ Wasserstoff, Methyl oder eine der Gruppen -CH₂R, -OR, -CH₂OR oder, sofern R² Methyl oder eine der Gruppen -CH₂R' bezeichnet, zusätzlich -CN, -COOH, -CO-OR, -CO-SR oder -O-CO-R bedeutet; R und R' für geradkettige oder verzweigte Alkylgruppen stehen; R¹ und R² gleiche oder verschiedene Bedeutung haben; und die Substituenten R¹ und R² einzeln bis zu 12 Kohlenstoffatome und zusammen höchstens 14 Kohlenstoffatome enthalten. Als Verwendung dieser mesogenen Verbindungen wird der Einsatz in Flüssigkristall-Mischungen beschrieben.

In Helvetica Chimica Acta, Vol. 65, Fasc. 4 (1982), Seiten 1242 - 1257, beschreiben M. Petrzilka und K. Schleich die Synthese von Alkyl-substituierten 2-Phenyl- und 2-Cyclohexyl-trans-Dekalinen sowie deren Eigenschaften. Die 2-Cyclohexyl-trans-Dekaline werden aus den entsprechenden 2-Phenyl-Cyclohexyl-trans-Dekalinen mit Alkylketon-Gruppe in 4'-Position durch Hydrierung, anschließender Jones-Oxidation mit Equilibrierung der erhaltenen trans-Ketone und schließlich Huang-Minlon Reduktion zur Alkyl-substituierten Verbindung hergestellt. Die so erhaltenen Verbindungen weisen breite Mesophasen-Bereiche bei hohen N-I-Übergangstemperaturen auf.

W. Sucrow und H. Wolter beschreiben in Chem. Ber. 118, (1985) 3350 - 3356 die Synthese des 6-(4'-Propyl-cyclohexyl)-trans-Dekalin-2-ol ausgehend vom 4-(trans-4'-Propylcyclohexyl)-Cyclohexanon. Zu den Estern und Ethern des Dekalin-2-ol wurden Übergangstemperaturen der mesogenen Phasen angegeben.

Die Druckschrift JP 2000 355557 A offenbart 2,6-Dekalinderivate mit weiteren Cyclohexanringen. Die Druckschrift DE 10112952 A offenbart ebensolche Dekalinverbindungen als Komponente eines flüssigkristallinen Mediums, wurde aber nach dem ersten Anmeldetag der vorliegenden Erfindung veröffentlicht.

Der vorliegenden Erfindung liegt die Aufgabe zugrunde, das Spektrum der für Flüssigkristall-Mischungen zur Verfügung stehenden 2,4'-substituierten 6-Cyclohexyl-trans-Dekaline zu erweitern, insbesondere Stoffe mit hoher dielektrischer Anisotropie bei niedriger Doppelbrechung zur Verfügung zu stellen. Darüber hinaus ist es eine Aufgabe dieser Erfindung Verwendungen solcher Verbindungen aufzuzeigen.

Die Erfindung wird gemäß Anspruch 1 mit 2,4'-substituierten 6-Cyclohexyl-trans-Dekalinen der Formel I gelöst.

Die erfindungsgemäßen Dekaline der Formel I weisen eine insbesondere für Flüssigkristall-Mischungen in elektrooptischen Anzeigeelementen wertvolle Kombination von hoher dielektrischer Anisotropie bei niedriger Doppelbrechung auf. In der Regel besitzen die erfindungsgemäßen Verbindungen Werte für die dielektrische Anisotropie Δε ≥ 4,0 und für die optische Anisotropie Δn ≤ 0,07. Darüber hinaus werden gegenüber bekannten Verbindungen mit vergleichbarer Grundstruktur vorteilhaft smektische Phasen unterdrückt. Zudem zeigen die erfindungsgemäßen Verbindungen eine gute Mischbarkeit mit anderen flüssigkristallinen Verbindungen.

R² besitzt folgende bevorzugte Bedeutung: H, Fluor, Chlor, -CN, Alkyl mit 1-12 C-Atomen, worin auch eine oder zwei nicht benachbarte CH₂-Gruppen durch -O-, -CO-, -O-CO-, -CO-O-, -E- und/ oder -C≡C- ersetzt sein können und/ oder worin auch ein oder mehrere H-Atome durch Fluor und/ oder Chlor ersetzt sein können.

Besonders bevorzugte Bedeutungen von R² sind H, Methyl, Ethyl, n-Propyl, n-Butyl, n-Pentyl, n-Hexyl, n-Heptyl, n-Octyl, Vinyl, 1-Propenyl, Allyl, 1-Butenyl, 2-Butenyl, 3-Butenyl, 3-Pentenyl, 4-Pentenyl, 1,5-Hexadienyl, Methoxy, Ethoxy, n-Propoxy, n-Butoxy, n-Pentoxy, n-Hexoxy, Vinyloxy, 1-Propenyloxy, 2-Propenyloxy, 2-Butenyloxy, Methoxy-methylen, 2-Methoxy-ethylen, 3-Methoxy-propylen, 4-Methoxy-butylen, 5-Methoxy-pentylen, Ethoxy-Methylen, 2-Ethoxy-Ethylen, Ethenoxy-Methylen, 2-Ethenoxy-Ethylen, worin auch ein oder mehrere H-Atome durch Fluor substituiert sein können.

Bevorzugt bedeutet R¹ und/oder R² unabhängig voneinander Alkyl, Alkoxy oder Oxaalkyl mit 1 bis 8 C-Atomen, wobei in R¹ mindestens ein H-Atom durch ein Halogen-Atom substituiert ist.

Falls R¹ und/oder R² einen Alkylrest bedeutet, so kann dieser geradkettig oder verzweigt sein. Vorzugsweise ist er geradkettig und bedeutet demnach insbesondere Methyl, Ethyl, n-Propyl, n-Butyl, n-Pentyl, n-Hexyl, n-Heptyl oder n-Octyl.

Falls R¹ und/oder R² einen Alkoxyrest bedeutet, so kann dieser geradkettig oder verzweigt sein. Vorzugsweise ist er geradkettig und bedeutet demnach insbesondere Methoxy, Ethoxy, n-Propoxy, n-Butoxy, n-Pentoxy, n-Hexoxy, n-Heptoxy oder n-Octoxy.

Falls R¹ und/oder R² einen Oxaalkylrest bedeutet, so kann dieser geradkettig oder verzweigt sein. Vorzugsweise ist er geradkettig und bedeutet demnach insbesondere 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl oder 2-, 3-, 4-, 5-, 6- oder 7-Oxaoctyl.

Bei den oben genannten Bedeutungen von R¹ als Alkyl, Alkoxy oder Oxaalkyl ist jeweils mindestens ein H-Atom durch ein Halogen-Atom, bevorzugt durch Fluor und/oder Chlor, besonders bevorzugt durch Fluor, substituiert. Bevorzugt sind 2 oder mehr H-Atome durch Fluor substituiert. Besonders bevorzugt sind in den oben angegebenen Resten in der endständigen Methyl-Gruppe 2 oder 3 H-Atome durch Fluor substituiert, so dass die oben angegebenen Reste eine CHF₂- oder eine CF₃-Gruppe aufweisen. Besonders bevorzugt ist der gesamte Rest R¹ perfluoriert.

Besonders bevorzugte fluorierte Reste R¹ als Alkyl sind -CHF₂, -CF₃, -CH₂CHF₂, -CH₂CF₃, -CF₂CHF₂, -CF₂CF₃, -CH₂CH₂CHF₂, -CH₂CH₂CF₃, -CH₂CF₂CHF₂, -CH₂CF₂CF₃, -CF₂CH₂CHF₂, -CF₂CH₂CF₃, -CF₂CF₂CHF₂, -CF₂CF₂CF₃, -C₂F₅, -C₃F₇, -C₄F₉, -C₅F₁₁, -C₆F₁₃, -C₇F₁₅ oder -C₈F₁₇.

Besonders bevorzugte fluorierte Reste R¹ als Alkoxy sind -OCHF₂, -OCF₃, -OCH₂CHF₂, -OCH₂CF₃, -OCF₂CHF₂, -OCF₂CF₃, -OCH₂CH₂CHF₂, -OCH₂CH₂CF₃, -OCH₂CF₂CHF₂, -OCH₂CF₂CF₃, -OCF₂CH₂CHF₂, -OCF₂CH₂CF₃, -OCF₂CF₂CHF₂, -OCF₂CF₂CF₃, -OC₄F₉, -OC₅F₁₁, -OC₆F₁₃, -OC₇F₁₅ oder -OC₈F₁₇.

Besonders bevorzugte fluorierte Reste R¹ als Oxaalkyl sind -CH₂OCHF₂, -CH₂OCF₃, -CF₂OCH₃, -CF₂OCHF₂, -CF₂OCF₃, -CH₂OCH₂CHF₂, -CH₂OCH₂CF₃, -CH₂OCF₂CF₃, -CF₂OCH₂CF₃ oder -CF₂OCF₂CF₃.

Z bedeutet vorzugsweise eine Einfachbindung, ferner -C₂F₄-.

Nachfolgend werden besonders bevorzugte erfindungsgemäße Verbindungen der Formel aufgerührt, wobei n Werte von 1 bis 6 annehmen kann:

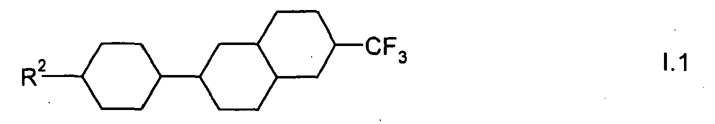

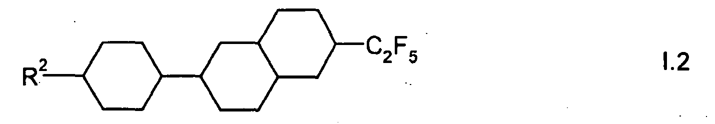

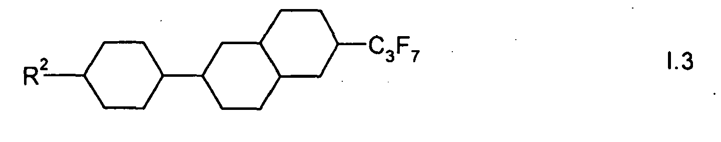

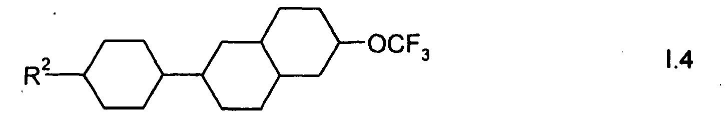

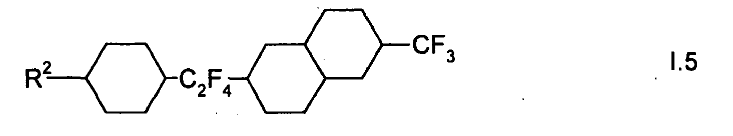

R² besitzt hierin eine der zuvor angegebenen Bedeutungen, insbesondere Methyl, Ethyl, n-Propyl, n-Butyl, n-Pentyl, n-Hexyl oder n-Heptyl.

Vor- und nachstehend sind Cyclohexylen-Gruppen in den erfindungsgemäßen Verbindungen sowie in weiteren, in flüssigkristallinen Medien eingesetzten Verbindungen vorzugsweise trans-ständig substituiert.

Die erfindungsgemäßen Verbindungen der Formel I, insbesondere der Formeln I.1 bis I.4, lassen sich vorteilhaft nach folgendem Schema herstellen.

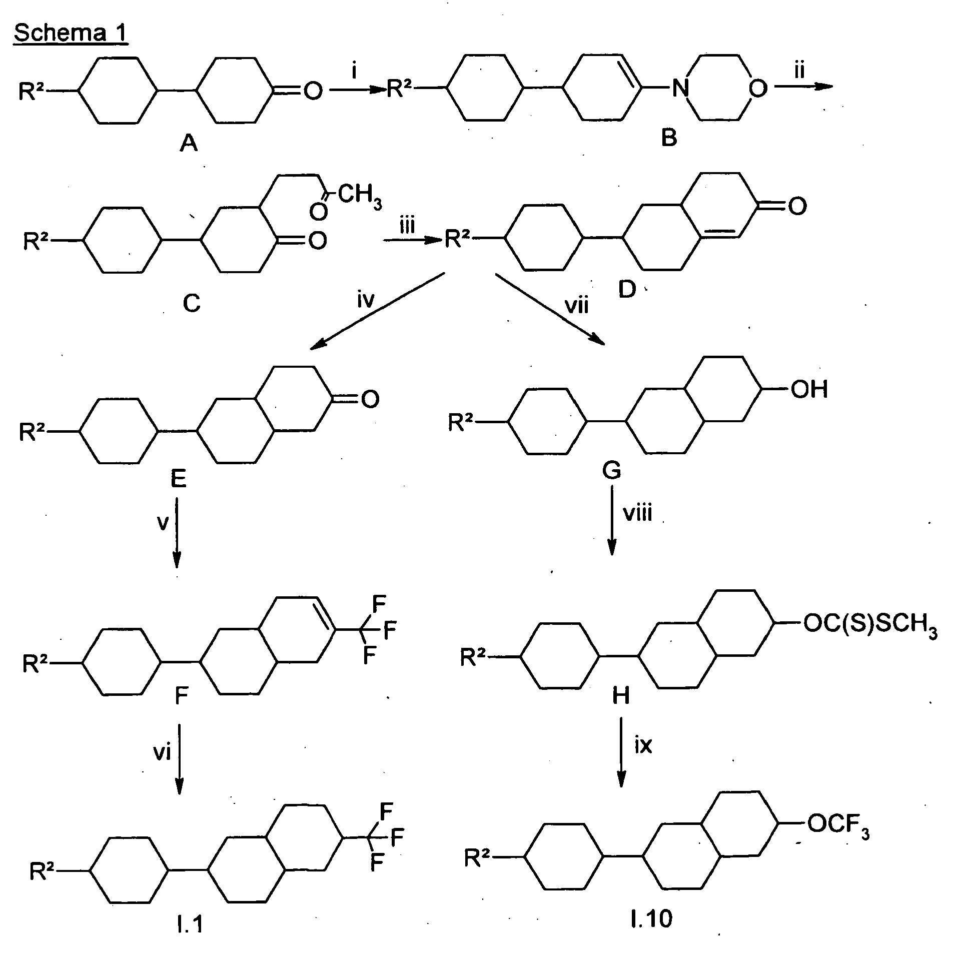

Verbindungen der Formel I mit einer -C₂F₄- Brücke können beispielsweise wie folgt hergestellt werden. 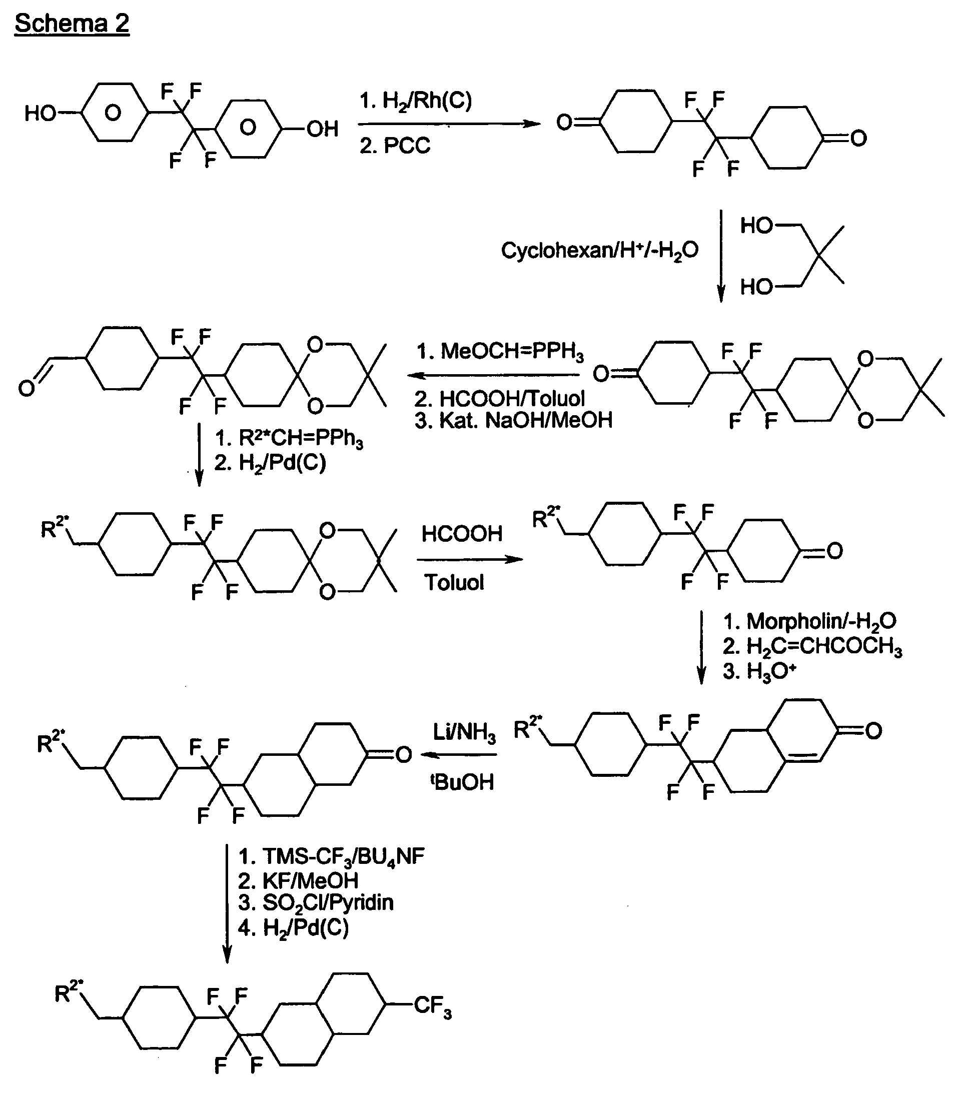
- R²*: in Schema 2 bedeutet H, Halogen, CN oder Alkyl mit 1 bis 17 C-Atomen, worin worin auch eine oder zwei nicht benachbarte -CH₂-Gruppen durch -O-, -S-, -CO-, -O-CO-, -CO-O- und/ oder -C=C- ersetzt sein können und/ oder worin auch eine oder mehrere H-Atome durch Halogen ersetzt sein können, bedeutet,

Ausgehend von dem trans-substituierten Cyclohexyl-Cyclohexanon der Formel A wird gemäß Schritt i unter Einsatz von Morpholin das entsprechende Enamin (B) gebildet, das gemäß Schritt ii mittels Methylvinylketon zur Keton-Verbindung der Formel C alkyliert wird. Im nachfolgenden Schritt iii wird eine Cyclisierung zum substituierten Dekalinenon der Formel D durchgeführt. Zur Bildung des gewünschten trans-Dekalin-Gerüsts durch Bildung des gewünschten Stereoisomeren der Verbindung C bei erhöhter Temperatur im sauren Medium und/ oder durch basenkatalysierte Cyclisierung wird auf die Ausführungen von W. Sucrow und H. Wolter (a. a. O.) verwiesen.

Ausgehend von dem Dekalinenon (D) kann über das entsprechende Dekalinon (E), das 4' substituierte 2-Trifluormethyl-6-Cyclohexyl-trans-Dekalin der Formel I.1 erhalten werden. Hierzu wird in Schritt iv eine Birch Reduktion durchgeführt, vorzugsweise mit Lithium in flüssigem Ammoniak und tert.-Butylalkohol und anschließender Jones-Oxidation des gebildeten sek. Alkohols. In Schritt v erfolgt die Umsetzung des Ketons E zunächst mit Trimethylsilyltrifluormethan und Tetrabutylammoniumfluorid, anschließender Zugabe von Kaliumfluorid in Methanol und abschließend Wasserentzug mittels Thionylchlorid (SOCl₂) in Pyridin. Schließlich wird in Schritt vi das gebildete Dekalinen (F) durch Hydrierung in Gegenwart von Palladium auf Aktivkohle zur gewünschten Verbindung 1.1 umgesetzt.

Alternativ hierzu kann ausgehend von dem Dekalinenon (D) über das Dekalinol (G), ferner über die Verbindung der Formel H das 4' substituierte 2-Trifluormethoxy-6-Cyclohexyl-trans-Dekalin der Formel I.10 erhalten werden. Im ersten Schritt vii wird mittels Birch-Reduktion in zwei Stufen nach W. Sucrow und H. Wolter (a.a.O) das trans-Dekalinol (G) erhalten. Die nachfolgenden beiden Schritte viii und ix werden gemäß der von Kiyoshi et al. in Chem. Lett. 1997, 827 - 828 am Beispiel der Umsetzung von Cyclohexanon zu Trifluormethoxy-Cyclohexanon beschriebenen Syntheseroute durchgeführt. Demnach wird gemäß Schritt viii durch Umsetzung mit einem Hydrierungsmittel, wie Natriumhydrid, Schwefelkohlenstoff (CS₂) und Dimethylsulfat die Verbindung H erhalten. Weitere Umsetzung mit Fluorwasserstoff in Pyridin und anschließend mit N-Bromsuccinimid (NBS) in Dichlormethan (CH₂Cl₂) führt zum gewünschten Trifluormethoxy-Dekalin (I.10).

Die für die oben angeführten Syntheseschritte jeweils geeigneten Reaktionsbedingungen, wie Druck, Temperatur, Reaktionszeiten und Lösungsmittel, sind dem Fachmann zumindest aus vergleichbaren Umsetzungen bekannt oder kann diese ohne weiteres aus vergleichbaren Umsetzungen ableiten und durch einfache Versuche optimieren. So sind beispielsweise die Bedingungen zur Durchführung einzelner Syntheseschritte zumindest vergleichbarer Umsetzungen bekannt durch W. Sucrow und H. Wolter (a. a. O.) und Kiyoshi et al. (a. a. O.).

Weitere erfindungsgemäße Verbindungen der Formel 1 können analog zu obigem Syntheseschema, gegebenenfalls unter Einbeziehung weiterer dem Fachmann bekannter Syntheseschritte, erhalten werden.

Alternativ zu obigem Syntheseschema sind die erfindungsgemäßen Verbindungen der Formel I auch über den Aufbau eines 2,4'-substituierten 6-Phenyl-trans-dekalin Gerüsts und anschließender Hydrierung zum entsprechenden 6-Cyclohexyl-trans-dekalin und gegebenenfalls weiterer Funktionalisierung zugänglich. Hierzu wird explizit auf die Veröffentlichungen von M. Petrzilka und K. Schleich (a. a. O. sowie DE 31 50 312 A1) verwiesen.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen, enantiotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäure-phenyl- oder cyclohexylester, Phenyl- oder Cyclohexyl-ester der Cyclohexylbenzoesäure, Phenyl- oder Cyclohexylester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢCH₃₋₍ₖ₊ₗ₎FₖClₗ, wobei i 0 oder 1 und k+l 1, 2 oder 3 sind; die Verbindungen, in denen R" diese Bedeutung hat, werden mit den Teilformeln 1 b, 2b, 3b, 4b und 5b bezeichnet. Besonders bevorzugt sind solche Verbindungen der Teilformeln 1 b, 2b, 3b, 4b und 5b, in denen R" die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1 a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise

| | |
|---|---|
| Gruppe A: | 0 bis 90 %, vorzugsweise 20 bis 90 %, insbesondere 30 bis 90 % |
| Gruppe B: | 0 bis 80 %, vorzugsweise 10 bis 80 %, insbesondere 10 bis 65 % |
| Gruppe C: | 0 bis 80 %, vorzugsweise 5 bis 80 %, insbesondere 5 bis 50 % |

wobei die Summe der Massenanteile der in den jeweiligen erfindungsgemäßen Medien enthaltenen Verbindungen aus den Gruppen A und/oder B und/oder C vorzugsweise 5 % bis 90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise zwei, drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/- R. Hatz, Handbook of Liquid Crystals, Verlag Chemie, Weinheim, 1980).

Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

In der vorliegenden Anmeldung und besonders in den folgenden Beispielen sind die Strukturen der flüssigkristallinen Verbindungen durch Abkürzungen, sogenannte Akronyme, wiedergegeben. Die Ableitung der entsprechenden Strukturen aus den Abkürzungen kann unmittelbar mittels der beiden folgenden Tabellen A und B erfolgen. Die Gruppen CₙH₂ₙ₊₁ und CₘH₂ₘ₊₁ sind geradkettige Alkylgruppen mit n bzw. m C-Atomen. Die Tabelle B ist selbsterklärend. Tabelle A beinhaltet lediglich die Abkürzungen für die Strukturen des Kemgerüsts. Die Einzelverbindungen werden durch die Abkürzung für den Kern, gefolgt von einem Bindestrich und einer Abkürzung für die Substituenten R¹, R², L¹ and L², gemäß folgender Liste, gekennzeichnet:

| Abkürzung für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| nO.Om | OCₙH₂ₙ₊₁ | OCₘH₂ₘ₊₁ | H | H |
| n | CₙH₂ₙ₊₁ | CN | H | H |
| nN.F | CₙH₂ₙ₊₁ | CN | H | F |
| nN.F.F | CₙH₂ₙ₊₁ | CN | F | F |
| nF | CₙH₂ₙ₊₁ | F | H | H |
| nF.F | CₙH₂ₙ₊₁ | F | H | F |
| nF.F.F | CₙH₂ₙ₊₁ | F | F | F |
| nOF | OCₙH₂ₙ₊₁ | F | H | H |
| nCl | CₙH₂ₙ₊₁ | Cl | H | H |
| nCl.F | CₙH₂ₙ₊₁ | Cl | H | F |
| nCl.F.F | CₙH₂ₙ₊₁ | Cl | F | F |
| nCF₃ | CₙH₂ₙ₊₁ | CF₃ | H | H |
| nCF₃.F | CₙH₂ₙ₊₁ | CF₃ | H | F |
| nCF₃.F.F | CₙH₂ₙ₊₁ | CF₃ | F | F |
| nOCF₃ | CₙH₂ₙ₊₁ | OCF₃ | H | H |
| nOCF₃.F | CₙH₂ₙ₊₁ | OCF₃ | H | F |
| nOCF₃.F.F | CₙH₂ₙ₊₁ | OCF₃ | F | F |
| nOCF₂ | CₙH₂ₙ₊₁ | OCHF₂ | H | H |
| nOCF₂.F | CₙH₂ₙ₊₁ | OCHF₂ | H | F |
| nOCF₂.F.F | CₙH₂ₙ₊₁ | OCHF₂ | F | F |
| nS | CₙH₂ₙ₊₁ | NCS | H | H |
| nS.F | CₙH₂ₙ₊₁ | NCS | H | F |
| nS.F.F | CₙH₂ₙ₊₁ | NCS | F | F |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |

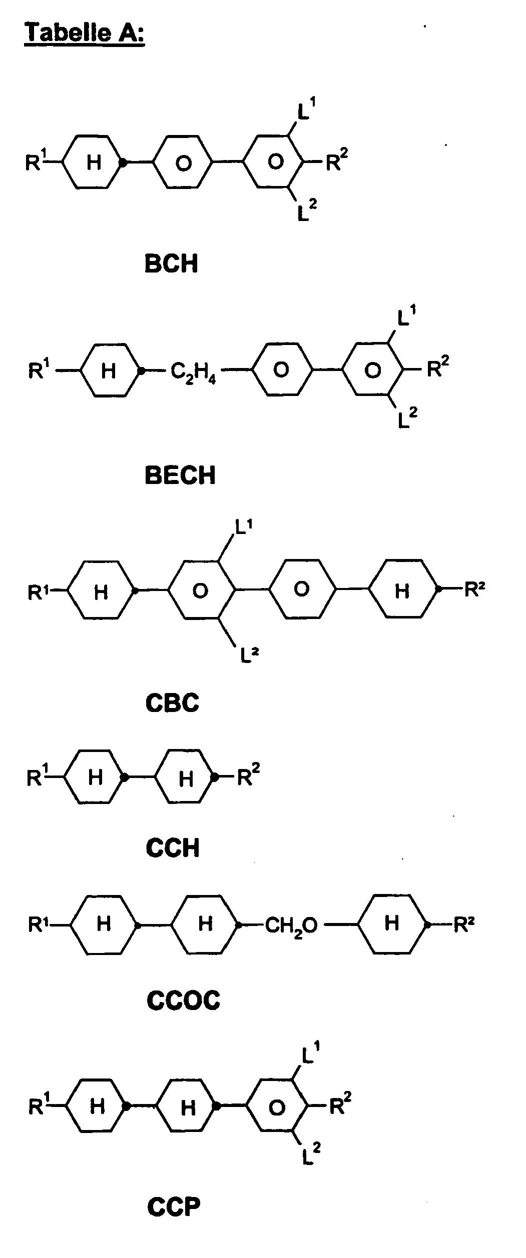

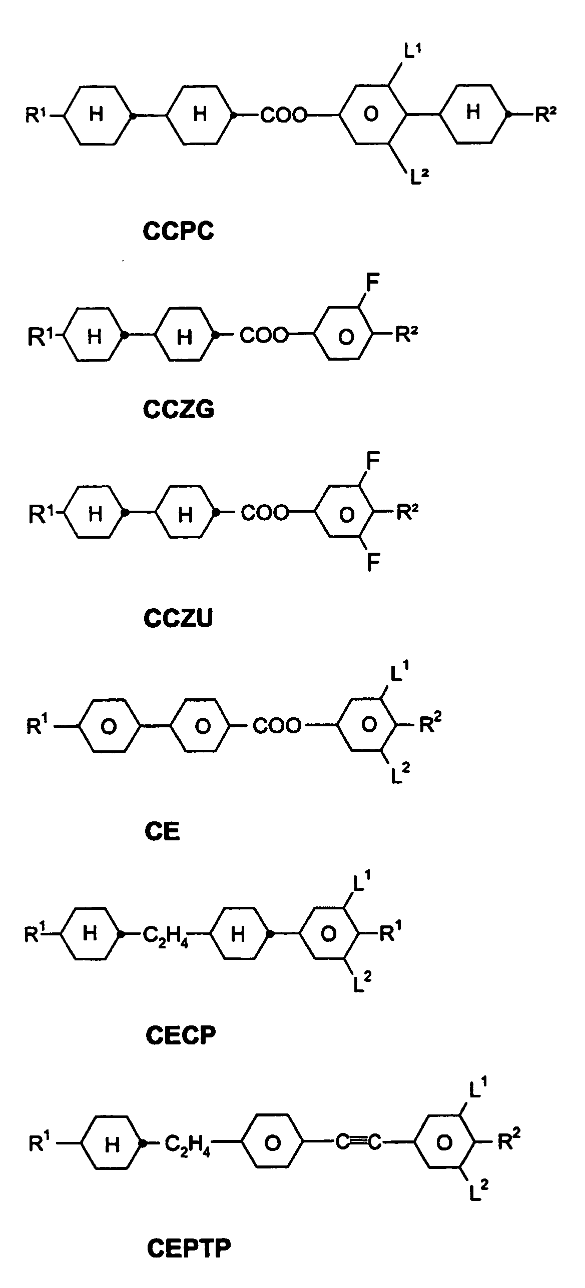

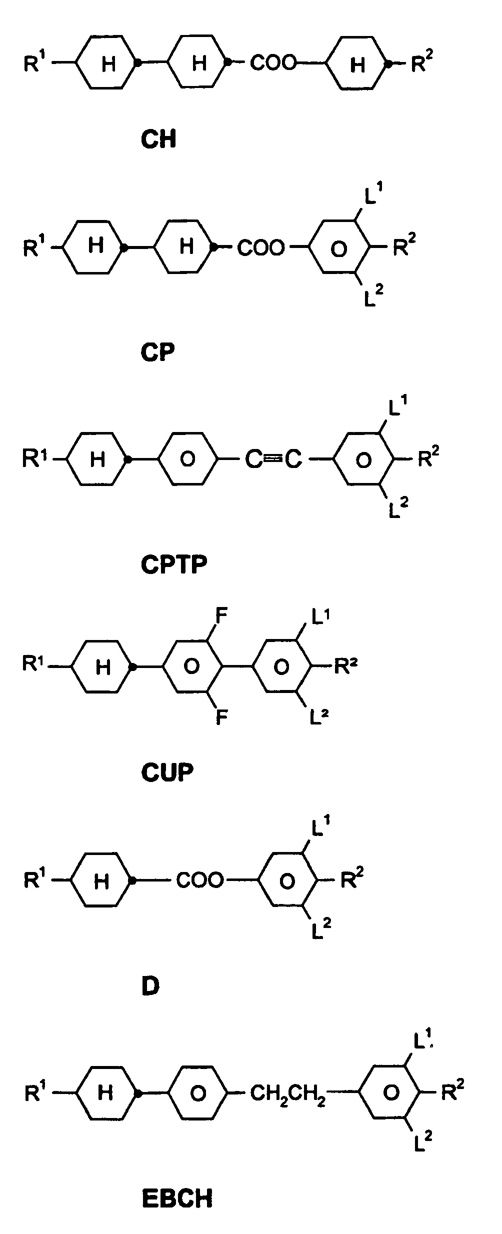

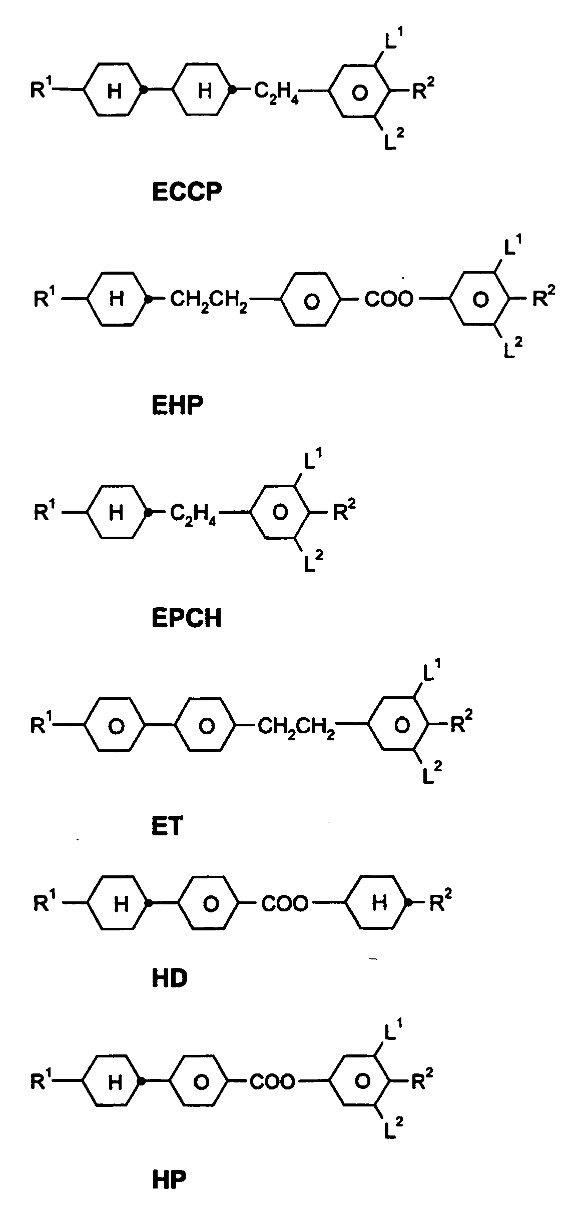

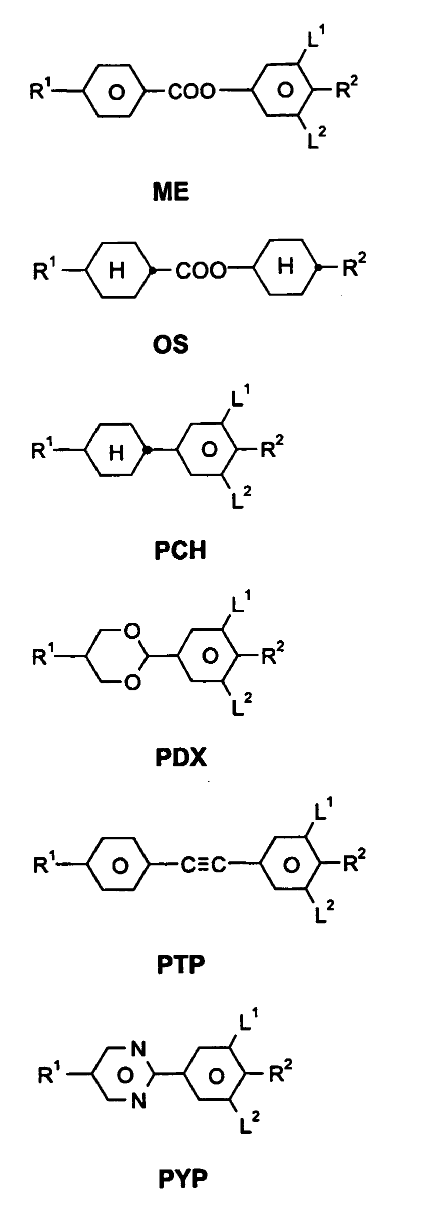

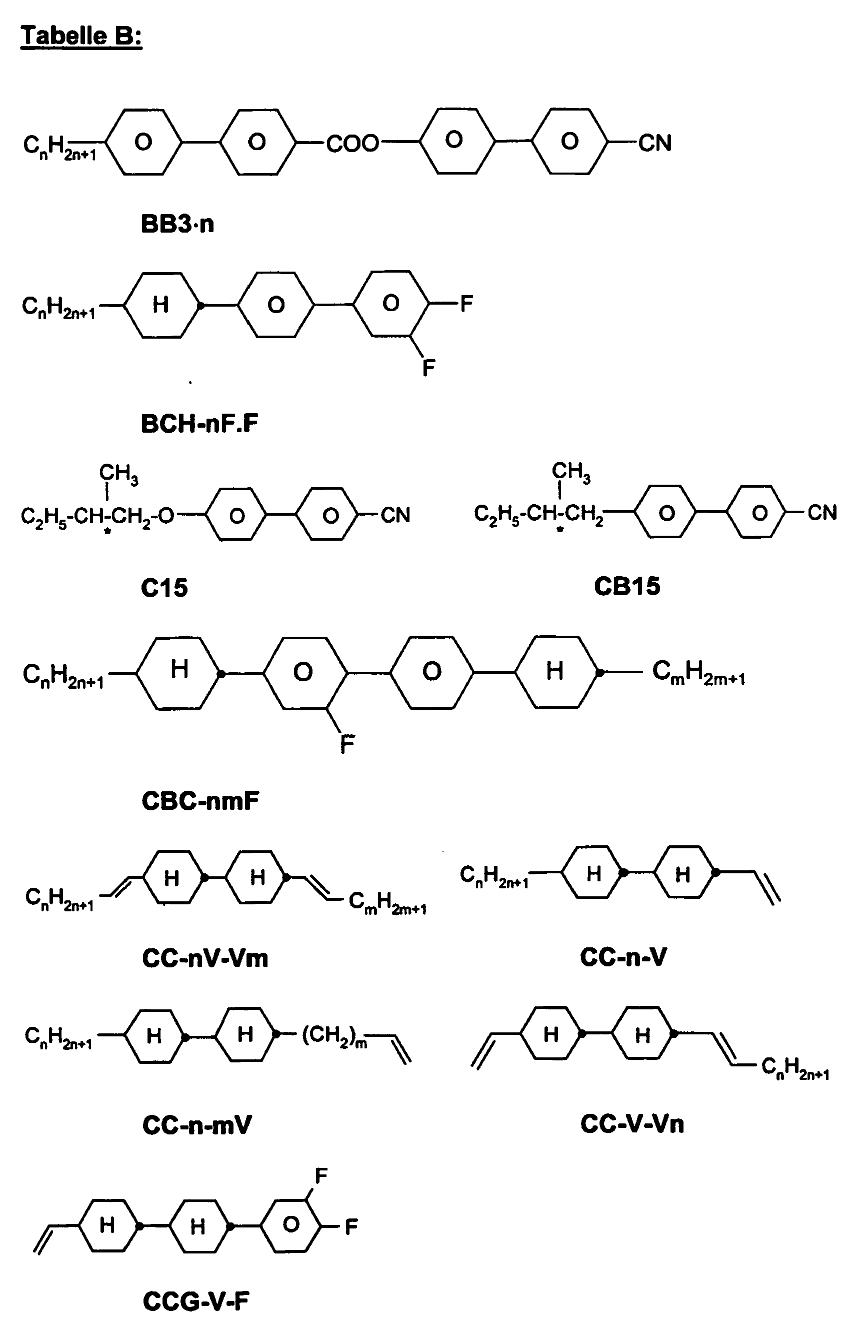

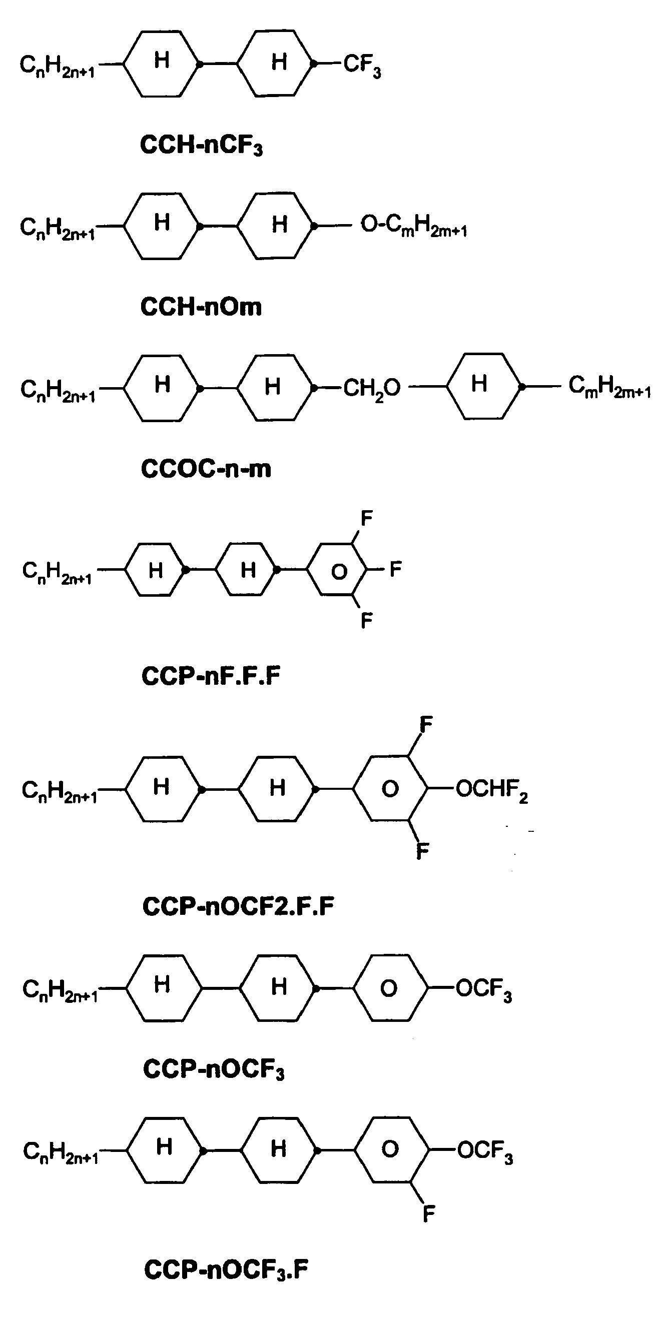

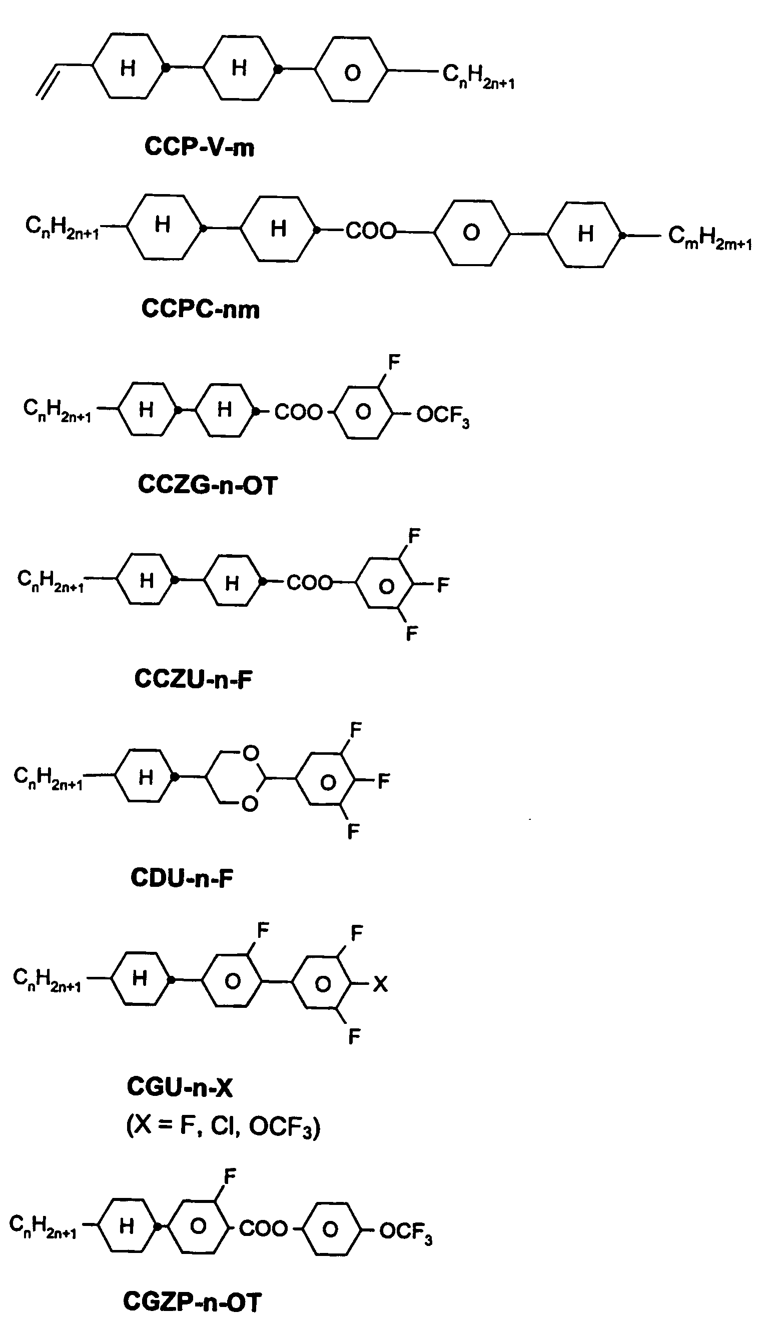

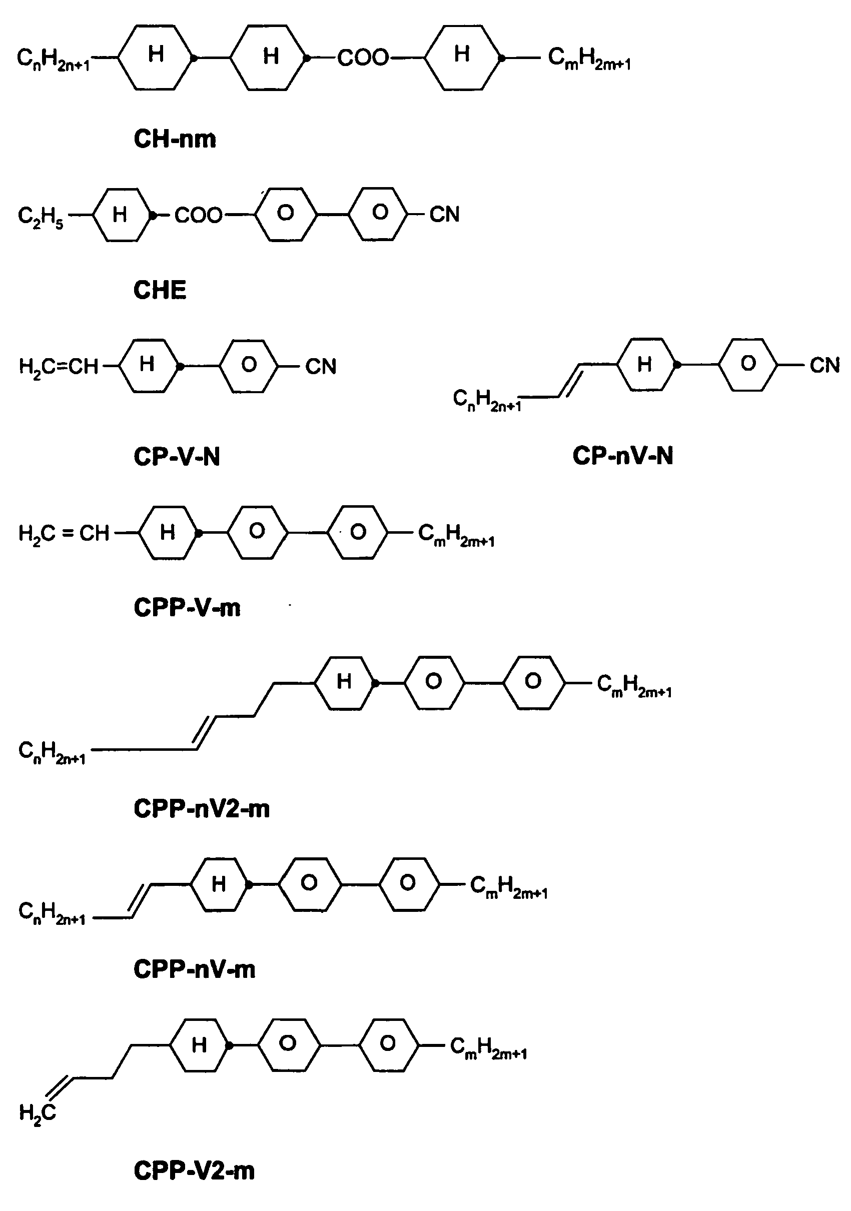

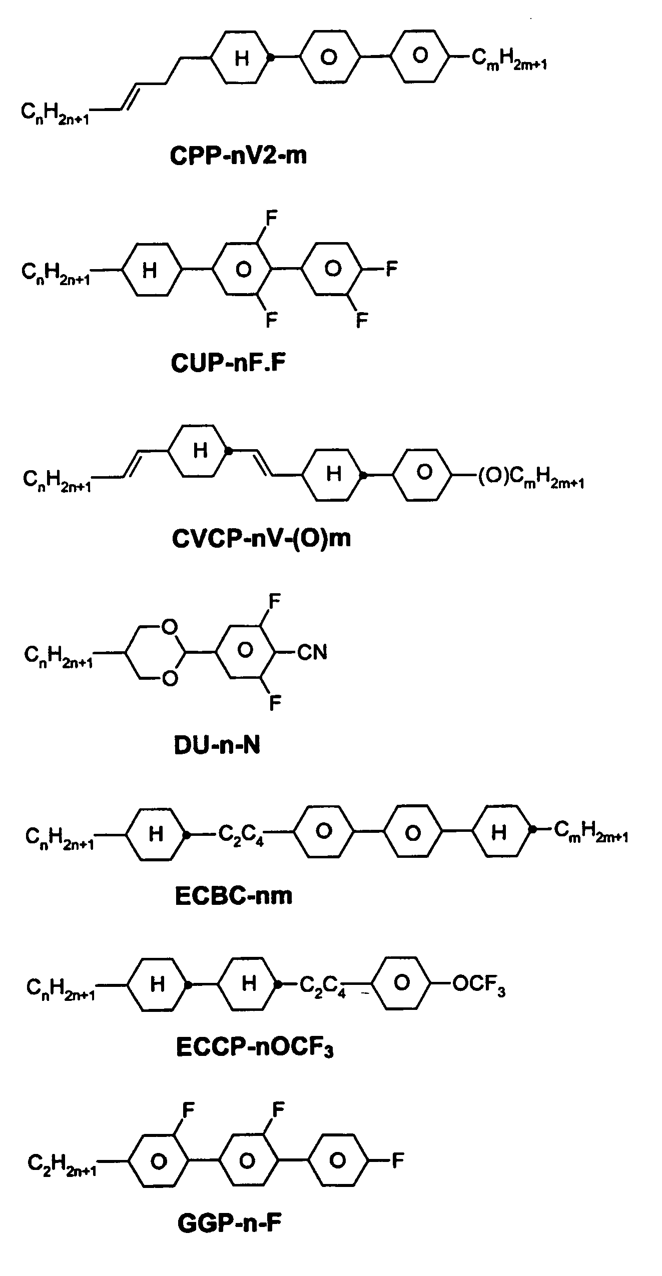

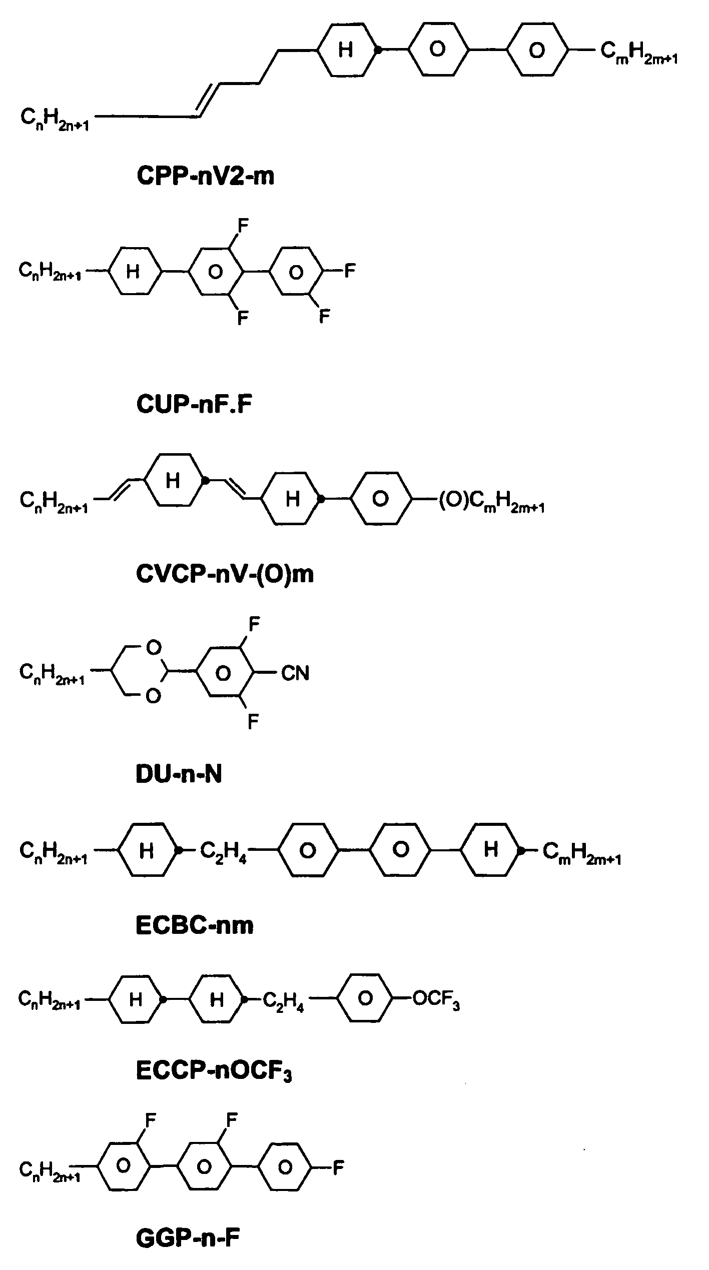

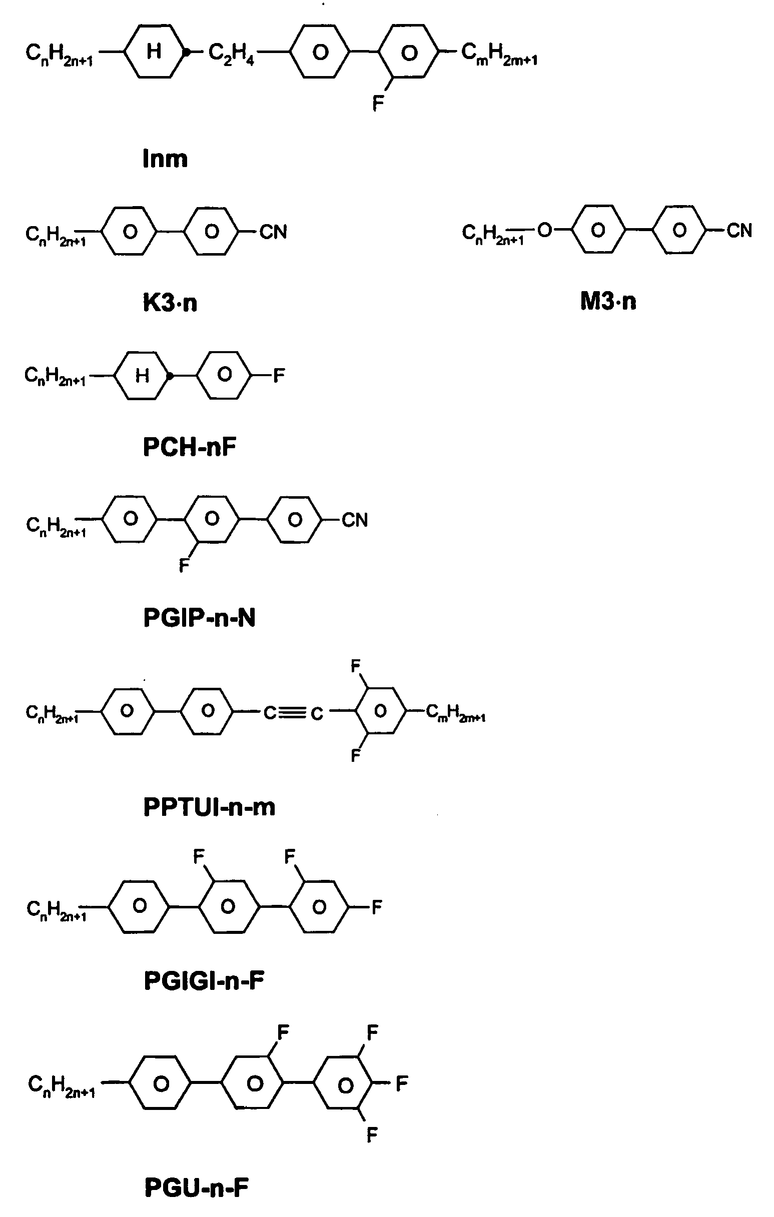

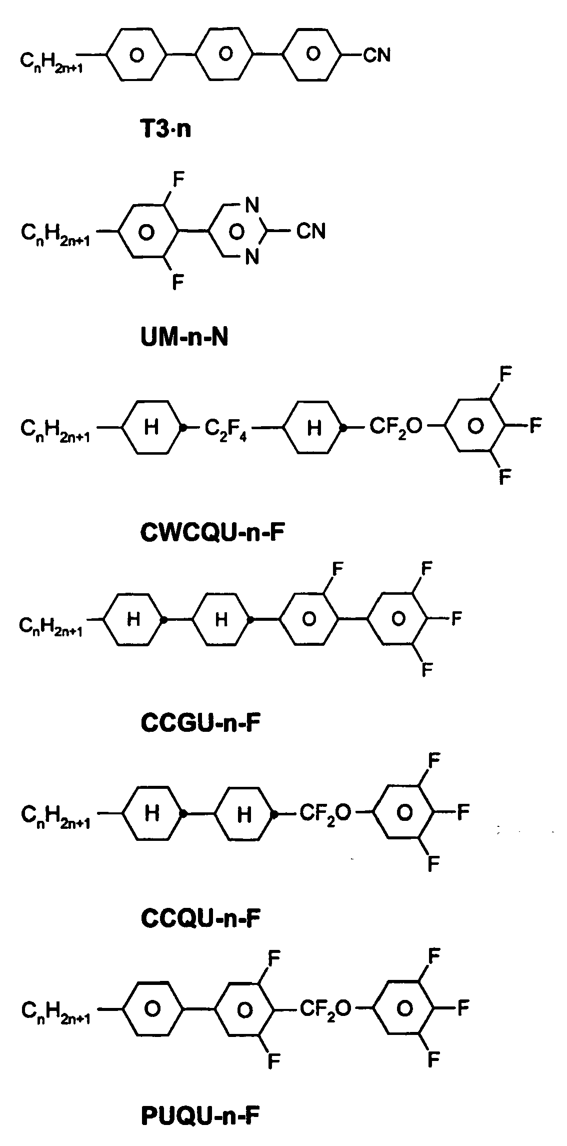

Die flüssigkristallinen Verbindungen der vorliegenden Erfindung enthalten vorzugsweise
- sieben oder mehr, vorzugsweise neun oder mehr Verbindungen ausgewählt aus der Gruppe der Verbindungen der Tabellen A und B und/oder
- vier oder mehr, vorzugsweise fünf oder mehr, Verbindungen ausgewählt aus der Gruppe der Verbindungen der Tabelle B und/oder
- fünf oder mehr, vorzugsweise sechs oder mehr, Verbindungen ausgewählt aus der Gruppe der Verbindungen der Tabelle A.

Solche erfindungsgemäßen Verbindungen und insbesondere die erfindungsgemäßen flüssigkristallinen Medien eignen sich vorteilhaft zur Anwendung in Flüssigkristall-Anzeigeelementen und als Dielektrikum in elektrooptischen Anzeigeelementen, wie beispielsweise TN-, STN- und Matrix-Flüssigkristallanzeigen, insbesondere solche mit einer sogenannten aktiven Matrix (AMD), wie TFT-Displays oder Displays mittels MOS (Metal Oxide Semiconductor)-Transistoren.

Die folgenden Ausführungsbeispiele sollen die Erfindung erläutern, ohne sie zu begrenzen. Vor- und nachstehend bedeuten Prozentangaben Gewichtsprozent. Alle Temperaturen sind in Grad Celsius angegeben.

In der vorangegangenen Liste sowie nachfolgend sind alle Temperaturen in Grad Celsius angegeben. Es bedeuten K = kristalliner Zustand, N = nematische Phase und I = isotrope Phase. Die Angaben zwischen diesen Symbolen stellen die Übergangstemperaturen dar. Δε bedeutet dielektrische Anisotropie (1 kHz, 20 °C), mit Δε = ε|| - ε_{┴}, wobei ε|| die Dielektrizitätskonstante parallel zu den Moleküllängsachsen und ε_{┴} die Dielektrizitätskonstante senkrecht hierzu bezeichnet. Δn bedeutet optische Anisotropie (589 nm, 20 °C) und n₀ den Brechungsindex. Die Fließviskosität ν₂₀ (mm2/sec) sowie die Rotationsviskosität γ₁ (mPa · s) wurden jeweils bei 20 °C bestimmt. V₁₀, V₅₀ und V₉₀ bezeichnen die Spannung für 10%, 50% bzw. 90% Transmission (Blickrichtung senkrecht zur Plattenoberfläche). Die elektrooptischen Daten wurden in einer TN-Zelle im 1. Minimum, d. h. bei einem d·Δn von 0,5, bei 20°C gemessen, sofern nicht ausdrücklich etwas anderes angegeben ist.

"Übliche Aufarbeitung" bedeutet: man gibt gegebenenfalls Wasser hinzu, stellt, falls erforderlich, je nach Konstitution des Endprodukts auf pH-Werte zwischen 2 und 10 ein, extrahiert mit einem geeigneten Lösungsmittel, beispielsweise Dichlormethan, Diethylether, Methyl-tert.-butylether oder Toluol, trennt ab, trocknet die organische Phase, dampft ein und reinigt das Produkt durch Destillation unter reduziertem Druck oder Kristallisation und/ oder Chromatographie.

### 1. Synthesebeispiele

### 1.1 Synthese von 2-Trifluormethyl-6-(4'-n-Propyl-cyclohexyl)-trans-Dekalin der Formel I.1.1

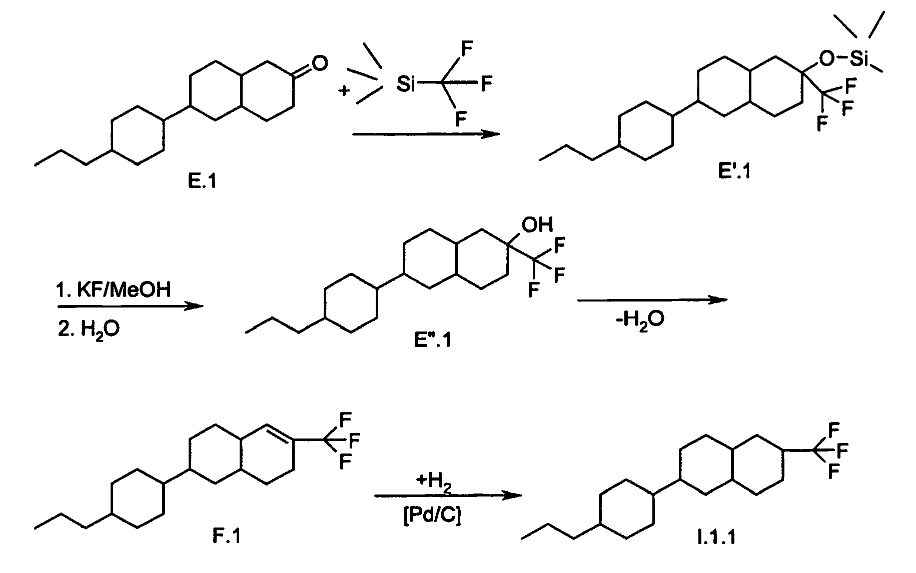

Unter Wasserausschluss werden 0,044 mol des Dekalinons der Formel E.1 mit 0,049 mol Trifluormethyltrimethylsilan in Tetrahydrofuran auf 0°C abgekühlt und 0,180 mol Tetrabutylammoniumfluorid zugetropft. Anschließend wird auf etwa 20°C erwärmen lassen und noch weitere 2 Stunden gerührt. Danach wird mit 20 ml 18%-iger Salzsäurelösung angesäuert und die organische Phase abgetrennt. Die wässrige Phase wird mit 50 ml Methyl-tert.-butylether extrahiert. Der vereinigte organische Extrakt wird mit Natriumchlorid-Lösung gewaschen, mit Natriumsulfat getrocknet und die Lösungsmittel entfernt.

0,043 mol des so erhaltenen Zwischenprodukts der Formel E'.1 werden in 100 ml Methanol gelöst, mit 500 mg Kaliumfluorid versetzt und über Nacht unter Rückfluss erhitzt und danach wie üblich aufgearbeitet.

Das erhaltene Zwischenprodukt der Formel E".1 wird in 70 ml Pyridin gelöst und bei 0°C langsam Thionylchlorid zugetropft. Das Reaktionsgemisch wird über Nacht gerührt. Anschließend wird wie üblich aufgearbeitet.

0,103 mol des Produkts aus der vorangegangenen Stufe der Formel F.1 werden in Tetrahydrofuran unter Zusatz von 9 g 5%-igem Palladium auf Aktivkohle hydriert. Es wird durch Entfernen des Lösungsmittels und Umkristallisieren das gewünschte Produkt I.1.1 erhalten. (K 66 N (61,2) I; Δε=5,0; Δn=0,0374; ν₂₀=38).

Analog werden die Verbindungen der Formel 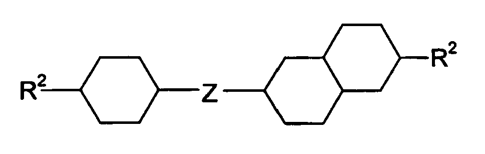 hergestellt.

| R¹ | R² | Z | |
|---|---|---|---|
| -CF₃ | H | - | |
| -CF₃ | -CH₃ | - | |
| -CF₃ | -C₂H₅ | - | |
| -CF₃ | -C₄H₉ | - | |
| -CF₃ | -C₅H₁₁ | - | K 91 I; Δε = 6,5; Δn=0,0420 |
| -CF₃ | -C₆H₁₃ | - | |
| -CF₃ | -C₇H₁₅ | - | |
| -C₂F₅ | -CH₃ | - | |
| -C₂F₅ | -C₂H₅ | - | |
| -C₂F₅ | -C₃H₇ | - | K 58 N 72,4 I; Δε = 5,8; Δn=0,034 |
| -C₂F₅ | -C₄H₉ | - | |
| -C₂F₅ | -C₅H₁₁ | - | |
| -C₂F₅ | -C₆H₁₃ | - | |
| -C₂F₅ | -C₇H₁₅ | - | |
| -C₃F₇ | -CH₃ | - | |
| -C₃F₇ | -C₂H₅ | - | |
| -C₃F₇ | -C₃H₇ | - | Δε = 4,8; Δn=0,034 |
| -C₃F₇ | -C₄H₉ | - | |
| -C₃F₇ | -C₅H₁₁ | - | |
| -C₃F₇ | -C₆H₁₃ | - | |
| -C₃F₇ | -C₇H₁₅ | - | |
| -CF₃ | H | | |
| -CF₃ | -CH₃ | -C₂F₄- | |
| -CF₃ | -C₂H₅ | -C₂F₄- | |
| -CF₃ | -C₃F₇ | -C₂F₄- | |
| -CF₃ | -C₄H₉ | -C₂F₄- | |
| -CF₃ | -C₅H₁₁ | -C₂F₄- | Δε = 3,4; Δn=0,033 |
| -CF₃ | -C₆H₁₃ | -C₂F₄- | |
| -CF₃ | -C₇H₁₅ | -C₂F₄- | |
| -C₂F₅ | -CH₃ | -C₂F₄- | |
| -C₂F₅ | -C₂H₅ | -C₂F₄- | |
| -C₂F₅ | ₋C₃H₇ | -C₂F₄- | |
| -C₂F₅ | -C₄H₉ | -C₂F₄- | |
| -C₂F₅ | -C₅H₁₁ | -C₂F₄- | |
| -C₂F₅ | -C₆H₁₃ | -C₂F₄- | |
| -C₂F₅ | -C₇H₁₅ | -C₂F₄- | |
| -C₃F₇ | -CH₃ | -C₂F₄- | |
| -C₃F₇ | -C₂H₅ | -C₂F₄- | |
| -C₃F₇ | -C₃H₇ | -C₂F₄- | |
| -C₃F₇ | -C₄H₉ | -C₂F₄- | |
| -C₃F₇ | -C₅H₁₁ | -C₂F₄- | |
| -C₃F₇ | -C₆H₁₃ | -C₂F₄- | |
| -C₃F₇ | -C₇H₁₅ | -C₂F₄- | |
| -OCF₃ | -CH₃ | | |
| -OCF₃ | -C₂H₅ | | |
| -OCF₃ | -C₄H₉ | | |
| -OCF₃ | -C₅H₁₁ | | |
| -OCF₃ | -C₆H₁₃ | | |
| -OCF₃ | -C₇H₁₅ | | |
| CF₂CF-O | -CH₃ | | |
| CF₂CF-O | -C₂H₅ | | |
| CF₂-CF-O | -C₃H₇ | | |
| CF₂-CF-O | -C₄H₉ | | |
| CF₂-CF-O | -C₅H₁₁ | | |
| CF₂-CF-O | -C₆H₁₃ | | |
| CF₂-CF-O | -C₇H₁₅ | | |
| CF₂-CF-O | -CH₃ | | |
| CF₂-CF-O | -C₂H₅ | | |
| CF₂-CF-O | -C₄H₉ | | |
| CF₂-CF-O | -C₅H₁₁ | | |
| CF₂-CF-O | -C₆H₁₃ | | |
| CF₂-CF-O | -C₇H₁₅ | | |

### Beispiel M1

| | | | |
|---|---|---|---|
| BCH-3F.F | 10,80 % | Klärpunkt [°C]: | 87,2 |
| BCH-5F.F | 9,00% | Δn [589 nm, 20 °C]: | 0,0906 |
| ECCP-3OCF₃ | 4,50 % | Δε [20 °C, 1 kHz]: | 5,2 |
| ECCP-5OCF₃ | 4,50% | ν₂₀ [mm²/s]: | 16 |
| CBC-33F | 1,80% | | |
| CBC-53F | 1,80% | | |
| CBC-55F | 1,80% | | |
| PCH-6F | 7,20 % | | |
| PCH-7F | 5,40 % | | |
| CCP-2OCF₃ | 7,20 % | | |
| CCP-3OCF₃ | 10,80 % | | |
| CCP-4OCF₃ | 6,30 % | | |
| CCP-5OCF₃ | 9,90% | | |
| PCH-5F | 9,00% | | |
| 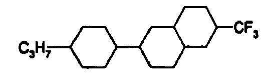 | 10,00 % | | |

### Beispiel M2

| | | | |
|---|---|---|---|
| CCH-3O1 | 11,20 % | Klärpunkt [°C]: | 73,9 |
| CCH-5O1 | 8,80% | Δn [589 nm, 20 °C]: | 0,0566 |
| CCP-2F.F.F | 8,00 % | Δε [20 °C, 1 kHz]: | 6,1 |
| CCP-3F.F.F | 10,40 % | | |
| CCP-5F.F.F | 4,00 % | | |
| CCZU-2-F | 4,00% | | |
| CCZU-3-F | 13,60 % | | |
| CCZU-5-F | 4,00 % | | |
| CH-33 | 2,40% | | |
| CH-35 | 2,40 % | | |
| CH-43 | 2,40 % | | |
| CCPC-33 | 2,40 % | | |
| CCH-3CF₃ | 6,40 % | | |
| 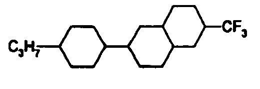 | 20,00 % | | |

### Beispiel M3

| | | | |
|---|---|---|---|
| PCH-5F | 3,20 % | Klärpunkt [°C]: | 98,1 |
| CCP-2OCF₂.F.F | 17,03 % | | |
| CCP-3OCF₂.F.F | 15,99 % | | |
| CCP-5OCF₂.F.F | 17,03 % | | |
| CUP-2F.F | 5,36% | | |
| CUP-3F.F | 5,36% | | |
| CBC-33F | 5,36% | | |
| CBC-53F | 5,36% | | |
| CBC-55F | 5,28 % | | |
| 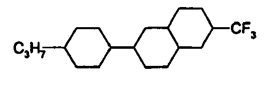 | 20,05 % | | |

### Beispiel M4

| | | | |
|---|---|---|---|
| CCP-2F.F.F | 11,0 % | Klärpunkt [°C]: | 79,0 |
| CCP-3F.F.F | 11,0 % | Δn [589 nm, 20 °C]: | 0,0720 |
| CCP-5F.F.F | 6,0 % | Δε [20 °C, 1 kHz]: | +5.5 |
| CCZU-2-F | 5,0% | V₁₀ [V]: | 1,37 |
| CCZU-3-F | 15,0% | V₅₀ [V]: | 1,68 |
| CCZU-5-F | 4,0% | V₉₀ [V]: | 2,11 |
| CCP-2OCF₃.F | 7,0% | | |
| CCP-5OCF₃.F | 5,0 % | | |
| CGU-2-F | 6,0% | | |
| CGU-3-F | 4,0 % | | |
| CCOC-3-3 | 2,0 % | | |
| CCOC-4-3 | 2,0% | | |
| CC-5-V | 9,0% | | |
| 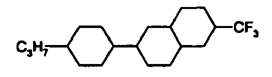 | 13,0% | | |

### Beispiel M5

| | | | |
|---|---|---|---|
| BCH-3F.F | 10,80 % | Klärpunkt [°C]: | 87,4 |
| BCH-5F.F | 9,00% | Δn [589 nm, 20 °C]: | 0,0915 |
| ECCP-3OCF₃ | 4,50 % | Δε [2.0 °C, 1 kHz]: | +5.5 |
| ECCP.5OCF₃ | 4,50 % | ν₂₀ [mm²/s¹] | 16 |
| CBC-33F | 1,80% | | |
| CBC-53F | 1,80 % | | |
| CBC-55F | 1,80 % | | |
| PCH-6F | 7,20% | | |
| PCH-7F | 5,40% | | |
| CCP-20CF₃ | 7,20 % | | |
| CCP-30CF₃ | 10,80 % | | |
| CCP-40CF₃ | 6,30% | | |
| CCP-50CF₃ | 9,90 % | | |
| PCH-5F | 9,00% | | |
| 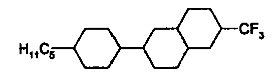 | 10,00 % | | |

### Beispiel M6:

| | |
|---|---|
| CCP-2F.F.F | 11,00 % |
| CCP-3F.F.F | 13,00 % |
| CCP-5F.F.F | 5,00 % |
| CCP-20CF₃.F | 7,00 % |
| CCP-30CF₃.F | 11,00 % |
| CGU-3-F | 4,00 % |
| CCOC-3-3 | 3,00% |
| CCOC-3-5 | 2,00 % |
| CCOC-4-3 | 4,00 % |
| CCH-3CF₃ | 8,00 % |
| CCH-5CF₃ | 7,00 % |
| 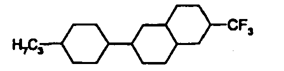 | 12,00 % |
| 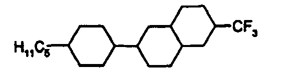 | 5,00% |
| CCGU-3-F | 8,00 % |

### Beispiel M7

| | |
|---|---|
| CCH-3CF₃ | 8,00 % |
| CCH-5CF₃ | 6,00 % |
| CCP-2F.F.F | 11,00 % |
| CCP-3F.F.F | 13,00 % |
| CCP-5F.F.F | 5,00 % |
| CCZU-2-F | 4,00 % |
| CCZU-3-F | 16,00 % |
| CCZU-5-F | 4,00 % |
| CCOC-33 | 3,00 % |
| CCOC-3-5 | 3,00 % |
| CCOC-4-3 | 4,00 % |
| 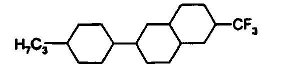 | 12,00 % |
| 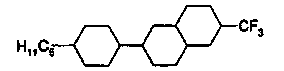 | 5,00 % |
| CCGU-3-F | 6,00 % |

### Beispiel M8

| | |
|---|---|
| CCP-2F.F.F | 11,00 % |
| CCP-3F.F.F | 13,00 % |
| CCP-5F.F.F | 6,00% |
| CCZU-2-F | 4,00% |
| CCZU-3-F | 16,00 % |
| CCZU-5-F | 4,00% |
| 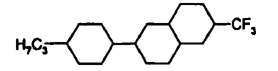 | 13,00 % |
| 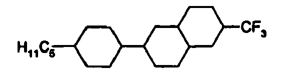 | 5,00 % |
| CCGU-2-F | 6,00 % |
| CCGU-3-F | 8,00 % |
| CCOC-3-3 | 3,00% |
| CCOC-3-5 | 2,00% |
| CCOC-4-5 | 3,00% |
| CCGU-3-F | 6,00% |

### Beispiel M9

| | | | |
|---|---|---|---|
| BCH-3F.F | 10,80 % | Klärpunkt [°C]: | +88,9 |
| BCH-5F.F | 9,00% | Δn [589 nm, 20 °C]: | 0,0908 |
| ECCP-30CF₃ | 4,50 % | Δε [20 °C, 1 kHz]: | +5.4 |
| ECCP.50CF₃ | 4,50 % | | |
| CBC-33F | 1,80% | | |
| CBC-53F | 1,80% | | |
| CBC-55F | 1,80% | | |
| PCH-6F | 7,20 % | | |
| PCH-7F | 5,40 % | | |
| CCP-20CF₃ | 7,20% | | |
| CCP-30CF₃ | 10,80 % | | |
| CCP-40CF₃ | 6,30% | | |
| CCP-50CF₃ | 9,90% | | |
| PCH-5F | 9,00% | | |
| 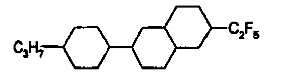 | 10,00 % | | |

### Beispiel 10:

| | | | |
|---|---|---|---|
| CCH-301 | 12,60 % | Klärpunkt [°C]: | +78,5 |
| CCH-3CF₃ | 7,20 % | Δn [589 nm, 20 °C]: | +0,0582 |
| CCH-501 | 9,90 % | Δε [20 °C, 1 kHz]: | +6,3 |
| CCP-2F.F.F | 9,00% | K₃/K₁: | 1,14 |
| CCP-3F.F.F | 11,70 % | | |
| CCP-5F.F.F | 4,50 % | | |
| CCPC-33 | 2,70% | | |
| CCZU-2-F | 4,50 % | | |
| CCZU-3-F | 15,30 % | | |
| CCZU-5-F | 4,50 % | | |
| CH-33 | 2,70 % | | |
| CH-35 | 2,70% | | |
| CH-43 | 2,70 % | | |
| 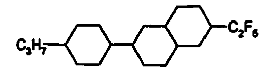 | 10,00 % | | |

### Beispiel 11

| | |
|---|---|
| CCH-301 | 4,00 % |
| CCH-501 | 10,00 % |
| CCP-1F.F.F | 6,00% |
| CCP-2F.F.F | 10,00 % |
| CCP-3F.F.F | 10,00 % |
| CCP-5F.F.F | 5,00% |
| CCOC-3-3 | 3,00% |
| CCOC-4-3 | 4,00 % |
| CCOC-3-5 | 2,00 % |
| CH-35 | 3,00 % |
| CWCQU-1-F | 7,00% |
| CWCQU-2-F | 7,00 % |
| CWCQU-3-F | 8,00% |
| CCZU-2-F | 5,00% |
| CCZU-3-F | 7,00% |
| 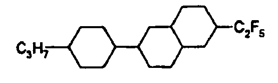 | 9,00% |

### Beispiel M12

| | |
|---|---|
| CCH-301 | 8,00 % |
| CCH-501 | 11,00 % |
| CCP-1F.F.F | 6,00% |
| CCP-2F.F.F | 10,00 % |
| CCP-3F.F.F | 10,00 % |
| CCP-5F.F.F | 6,00% |
| CCP-3OCF₃.F | 6,00 % |
| CCP-5OCF₃.F | 4,00 % |
| CCOC-3-3 | 2,00 % |
| CCOC-3-5 | 2,00% |
| CCOC-4-3 | 3,00 % |
| CWCQU-1-F | 6,00 % |
| CWCQU-2-F | 6,00% |
| CWCQU-3-F | 7,00 % |
| 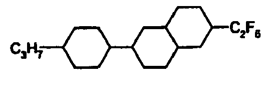 | 13,00 % |

### Beispiel M 13

| | |
|---|---|
| CCH-301 | 10,00 % |
| CCH-501 | 13,00 % |
| CCP-1F.F.F | 7,00% |
| CCP-2F.F.F | 10,00 % |
| CCP-3F.F.F | 10,00 % |
| CCP-5F.F.F | 5,00% |
| CCP-20CF₃.F | 5,00% |
| CCP-30CF₃.F | 9,00% |
| CCP-50CF₃.F | 10,00 % |
| CCOC-3-3 | 3,00 % |
| CCOC-3-5 | 2,00 % |
| CCOC-4-3 | 4,00% |
| 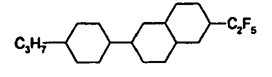 | 12,00 % |

### Beispiel M14

| | |
|---|---|
| CCH-301 | 4,00 % |
| CCH-501 | 5,00 % |
| CCH-3CF₃ | 3,00% |
| CCH-5CF₃ | 5,00 % |
| CCP-1F.F.F | 9,00% |
| CCP-2F.F.F | 11,00% |
| CCP-3F.F.F | 10,00 % |
| CCP-5F.F.F | 5,00 % |
| CCP-30CF₃.F | 10,00 % |
| CCP-50CF₃.F | 10,00 % |
| CCOC-3-3 | 3,00 % |
| CCOC-3-5 | 3,00 % |
| CCOC-4-3 | 4,00 % |
| 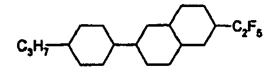 | 18,00 % |

## Patentansprüche

1. 2,4'-substituierte 6-Cyclohexyl-trans-Dekaline der Formel I 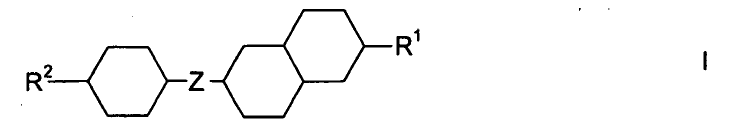 worin
R¹ Halogen oder Alkyl mit 1 bis 18 C-Atomen, worin auch eine oder zwei nicht benachbarte CH₂-Gruppen durch -O-, -S-, -CO-, -O-CO-, -CO-O, und/ oder -C≡C-ersetzt sein können, und worin mindestens ein H-Atom durch ein Halogen-Atom substituiert ist, bedeutet,
R² H, Halogen, -CN oder Alkyl mit 1 bis 18 C-Atomen, worin auch eine oder zwei nicht benachbarte -CH₂-Gruppen durch -O-, -S-, -CO-, -O-CO-, -CO-O und/oder -C≡C- ersetzt sein können und/ oder worin auch eine oder mehrere H-Atome durch Halogen ersetzt sein können, bedeutet, und
Z Einfachbindung, -CH₂O-, -OCH₂-, -COO-, -C≡C-, -CH=CH-, -CF₂O-, -OCF₂-, -CF=CF-, -C₂F₄-, -CH=CH-(CH₂)₂- oder -(CH₂)₄-,
bedeutet.

2. 2,4'-substituierte 6-Cyclohexyl-trans-Dekaline der Formel I nach Anspruch 1, **dadurch gekennzeichnet, dass** R² H, Methyl, Ethyl, n-Propyl, n-Butyl, n-Pentyl, n-Hexyl, n-Heptyl oder n-Octyl bedeutet.

3. 2,4'-substituierte 6-Cyclohexyl-trans-Dekaline der Formel I nach Anspruch 1 oder 2, **dadurch gekennzeichnet, dass** R¹ zwei oder mehr F-Atome aufweist.

4. 2,4'-substituierte 6-Cyclohexyl-trans-Dekaline der Formel I nach einem der Ansprüche 1 bis 3, **dadurch gekennzeichnet, dass** R¹ perfluoriert ist.

5. 2,4'-substituierte 6-Cyclohexyl-trans-Dekaline der Formel I nach einem der Ansprüche 1 bis 4, **dadurch gekennzeichnet, dass** R¹ und/oder R² unabhängig voneinander Alkyl, Alkoxy oder Oxaalkyl mit 1 bis 8 C-Atomen bedeutet, wobei in R¹ mindestens ein H-Atom durch ein Halogen-Atom substituiert ist.

6. 2,4'-substituierte 6-Cyclohexyl-trans-Dekaline der Formel I nach einem der Ansprüche 1 bis 3, **dadurch gekennzeichnet, dass** R¹ und/oder R² unabhängig voneinander -CF₃, -C₂F₅, -C₃F₇ oder -OCF₃ bedeutet, worin n einen Wert von 1 bis 6 besitzt.

7. Verwendung von Verbindungen der Formel I nach einem der Ansprüche 1 bis 6 als Komponenten flüssigkristalliner Medien.

8. Flüssigkristallines Medium mit zwei oder mehreren flüssigkristallinen Komponenten, **dadurch gekennzeichnet, dass** es mindestens eine Verbindung der Formel I nach einem der Ansprüche 1 bis 6 enthält.

9. Flüssigkristall-Anzeigeelement, **dadurch gekennzeichnet, dass** es ein flüssigkristallines Medium nach Anspruch 8 enthält.

10. Elektrooptisches Anzeigeelement, **dadurch gekennzeichnet, dass** es als Dielektrikum ein flüssigkristallines Medium nach Anspruch 8 enthält.

## Patentansprüche (Patentansprüche für folgende(n) Vertragsstaat(en): DE)

1. 2,4'-substituierte 6-Cyclohexyl-trans-Dekaline der Formel I 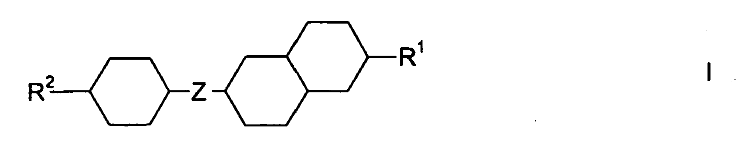 worin
R¹ Halogen oder Alkyl mit 1 bis 18 C-Atomen, worin auch eine oder zwei nicht benachbarte CH₂-Gruppen durch -O-, -S-, -CO-, -O-CO-, -CO-O und/ oder -C≡C-ersetzt sein können, und worin mindestens ein H-Atom durch ein Halogen-Atom substituiert ist, bedeutet,
R² H, Halogen, -CN oder Alkyl mit 1 bis 18 C-Atomen, worin auch eine oder zwei nicht benachbarte -CH₂-Gruppen durch -O-, -S-, -CO-, -O-CO-, -CO-O und/oder -C=C- ersetzt sein können und/ oder worin auch eine oder mehrere H-Atome durch Halogen ersetzt sein können, bedeutet, und
Z -CH₂O-, -OCH₂-, -COO-, -C≡C-, -CH=CH-, -CF₂O-, -OCF₂-, -CF=CF-, -C₂F₄-, -CH=CH-(CH₂)₂- oder -(CH₂)₄-,
bedeutet.

2. 2,4'-substituierte 6-Cyclohexyl-trans-Dekaline der Formel I nach Anspruch 1, **dadurch gekennzeichnet, dass** R² H, Methyl, Ethyl, n-Propyl, n-Butyl, n-Pentyl, n-Hexyl, n-Heptyl oder n-Octyl bedeutet.

3. 2,4'-substituierte 6-Cyclohexyl-trans-Dekaline der Formel I nach Anspruch 1 oder 2, **dadurch gekennzeichnet, dass** R¹ zwei oder mehr F-Atome aufweist.

4. 2,4'-substituierte 6-Cyclohexyl-trans-Dekaline der Formel I nach einem der Ansprüche 1 bis 3, **dadurch gekennzeichnet, dass** R¹ perfluoriert ist.

5. 2,4'-substituierte 6-Cyclohexyl-trans-Dekaline der Formel I nach einem der Ansprüche 1 bis 4, **dadurch gekennzeichnet, dass** R¹ und/oder R² unabhängig voneinander Alkyl, Alkoxy oder Oxaalkyl mit 1 bis 8 C-Atomen bedeutet, wobei in R¹ mindestens ein H-Atom durch ein Halogen-Atom substituiert ist.

6. 2,4'-substituierte 6-Cyclohexyl-trans-Dekaline der Formel I nach einem der Ansprüche 1 bis 3, **dadurch gekennzeichnet, dass** R¹ und/oder R² unabhängig voneinander -CF₃, -C₂F₅, -C₃F₇ oder -OCF₃ bedeutet, worin n einen Wert von 1 bis 6 besitzt.

7. Verwendung von Verbindungen der Formel I nach einem der Ansprüche 1 bis 6 als Komponenten flüssigkristalliner Medien.

8. Flüssigkristallines Medium mit zwei oder mehreren flüssigkristallinen Komponenten, **dadurch gekennzeichnet, dass** es mindestens eine Verbindung der Formel I nach einem der Ansprüche 1 bis 6 enthält.

9. Flüssigkristall-Anzeigeelement, **dadurch gekennzeichnet, dass** es ein flüssigkristallines Medium nach Anspruch 8 enthält.

10. Elektrooptisches Anzeigeelement, **dadurch gekennzeichnet, dass** es als Dielektrikum ein flüssigkristallines Medium nach Anspruch 8 enthält.

## Claims

1. 2,4'-Substituted 6-cyclohexyl-trans-decalins of the formula I 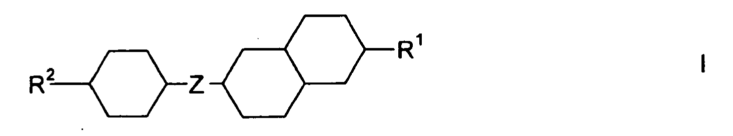 in which
R¹ denotes halogen or alkyl having 1 to 18 C atoms, in which, in addition, one or two non-adjacent CH₂ groups may be replaced by -O-, -S-, -CO-, -O-CO-, -CO-O- and/or -C≡C- and in which at least one H atom is substituted by a halogen atom,
R² denotes H, halogen, -CN or alkyl having 1 to 18 C atoms, in which, in addition, one or two non-adjacent CH₂ groups may be replaced by -O-, -S-, -CO-, -O-CO-, -CO-O- and/or -C≡C- and/or in which, in addition, one or more H atoms may be replaced by halogen, and
Z denotes single bond, -CH₂O-, -OCH₂-, -COO-, -C≡C-, -CH=CH-, -CF₂O- -OCF₂-, -CF=CF-, -C₂F₄-, -CH=CH-(CH₂)₂- or -(CH₂)₄-.

2. 2,4'-Substituted 6-cyclohexyl-trans-decalins of the formula I according to Claim 1, **characterised in that** R² denotes H, methyl, ethyl, n-propyl, n-butyl, n-pentyl, n-hexyl, n-heptyl or n-octyl.

3. 2,4'-Substituted 6-cyclohexyl-trans-decalins of the formula I according to Claim 1 or 2, **characterised in that** R¹ contains two or more F atoms.

4. 2,4'-Substituted 6-cyclohexyl-trans-decalins of the formula I according to one of Claims 1 to 3, **characterised in that** R¹ is perfluorinated.

5. 2,4'-Substituted 6-cyclohexyl-trans-decalins of the formula I according to one of Claims 1 to 4, **characterised in that** R¹ and/or R², independently of one another, denote alkyl, alkoxy or oxaalkyl having 1 to 8 C atoms, where at least one H atom in R¹ is substituted by a halogen atom.

6. 2,4'-Substituted 6-cyclohexyl-trans-decalins of the formula I according to one of Claims 1 to 3, **characterised in that** R¹ and/or R², independently of one another, denote -CF₃, -C₂F₅, -C₃F₇ or -OCF₃, in which n has a value of 1 to 6.

7. Use of compounds of the formula I according to one of Claims 1 to 6 as components of liquid-crystalline media.

8. Liquid-crystalline medium having two or more liquid-crystalline components, **characterised in that** it comprises at least one compound of the formula I according to one of Claims 1 to 6.

9. Liquid-crystal display element, **characterised in that** it contains a liquid-crystalline medium according to Claim 8.

10. Electro-optical display element, **characterised in that** it contains, as dielectric, a liquid-crystalline medium according to Claim 8.

## Claims (Claims for the following Contracting State(s): DE)

1. 2,4'-Substituted 6-cyclohexyl-trans-decalins of the formula I 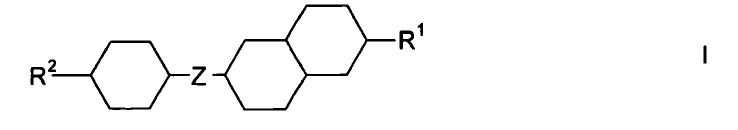 in which
R¹ denotes halogen or alkyl having 1 to 18 C atoms, in which, in addition, one or two non-adjacent CH₂ groups may be replaced by -O-, -S-, -CO-, -O-CO-, -CO-O- and/or -C≡C- and in which at least one H atom is substituted by a halogen atom,
R² denotes H, halogen, -CN or alkyl having 1 to 18 C atoms, in which, in addition, one or two non-adjacent CH₂ groups may be replaced by -O-, -S-, -CO-, -O-CO-, -CO-O- and/or -C≡C- and/or in which, in addition, one or more H atoms may be replaced by halogen, and
Z denotes -CH₂O-, -OCH₂-, -COO-, -C≡C-, -CH=CH-, -CF₂O-, -OCF₂-, -CF=CF-, -C₂F₄-, -CH=CH-(CH₂)₂- or -(CH₂)₄-.

2. 2,4'-Substituted 6-cyclohexyl-trans-decalins of the formula I according to Claim 1, **characterised in that** R² denotes H, methyl, ethyl, n-propyl, n-butyl, n-pentyl, n-hexyl, n-heptyl or n-octyl.

3. 2,4'-Substituted 6-cyclohexyl-trans-decalins of the formula I according to Claim 1 or 2, **characterised in that** R¹ contains two or more F atoms.

4. 2,4'-Substituted 6-cyclohexyl-trans-decalins of the formula I according to one of Claims 1 to 3, **characterised in that** R¹ is perfluorinated.

5. 2,4'-Substituted 6-cyclohexyl-trans-decalins of the formula I according to one of Claims 1 to 4, **characterised in that** R¹ and/or R², independently of one another, denote alkyl, alkoxy or oxaalkyl having 1 to 8 C atoms, where at least one H atom in R¹ is substituted by a halogen atom.

6. 2,4'-Substituted 6-cyclohexyl-trans-decalins of the formula I according to one of Claims 1 to 3, **characterised in that** R¹ and/or R², independently of one another, denote -CF₃, -C₂F₅, -C₃F₇ or -OCF₃, in which n has a value of 1 to 6.

7. Use of compounds of the formula I according to one of Claims 1 to 6 as components of liquid-crystalline media.

8. Liquid-crystalline medium having two or more liquid-crystalline components, **characterised in that** it comprises at least one compound of the formula I according to one of Claims 1 to 6.

9. Liquid-crystal display element, **characterised in that** it contains a liquid-crystalline medium according to Claim 8.

10. Electro-optical display element, **characterised in that** it contains, as dielectric, a liquid-crystalline medium according to Claim 8.

## Revendications

1. 6-Cyclohexyle-trans-décalines substituées-2,4' de la formule I 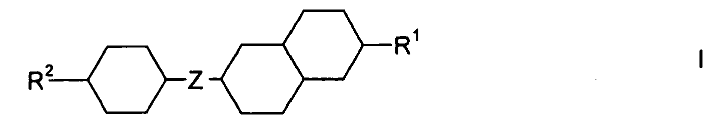 dans laquelle
R¹ représente halogène ou alkyle ayant 1 à 18 atomes de C, dans lequel, en addition, un ou deux groupes CH₂ non adjacents peuvent être remplacés par -O-, -S-, -CO-, -O-CO-, -CO-O- et/ou -C≡C- et dans lequel au moins un atome de H est substitué par un atome d'halogène,
R² représente H, halogène, -CN ou alkyle ayant 1 à 18 atomes de C, dans lequel, en addition, un ou deux groupes CH₂ non adjacents peuvent être remplacés par -O-, -S-, -CO-, -O-CO-, -CO-O- et/ou -C≡C- et/ou dans lequel, en addition, un ou plusieurs atomes de H peuvent être remplacés par halogène, et
Z représente une liaison simple, -CH₂O-, -OCH₂-, -COO-, -C≡C-, -CH=CH-, -CF₂O-, -OCF₂-, -CF=CF-, -C₂F₄-, -CH=CH-(CH₂)₂- ou -(CH₂)₄-.

2. 6-Cyclohexyle-trans-décalines substituées-2,4' de la formule I selon la revendication 1, **caractérisées en ce que** R² représente H, méthyle, éthyle, n-propyle, n-butyle, n-pentyle, n-hexyle, n-heptyle ou n-octyle.

3. 6-Cyclohexyle-trans-décalines substituées-2,4' de la formule I selon la revendication 1 ou 2, **caractérisées en ce que** R¹ contient deux ou plus de deux atomes de F.

4. 6-Cyclohexyle-trans-décalines substituées-2,4' de la formule I selon l'une des revendications 1 à 3, **caractérisées en ce que** R¹ est perfluoré.

5. 6-Cyclohexyle-trans-décalines substituées-2,4' de la formule I selon l'une des revendications 1 à 4, **caractérisées en ce que** R¹ et/ou R², indépendamment l'un de l'autre, représentent alkyle, alcoxy ou oxaalkyle ayant 1 à 8 atomes de C, où au moins un atome de H dans R¹ est substitué par un atome d'halogène.

6. 6-Cyclohexyle-trans-décalines substituées-2,4' de la formule I selon l'une des revendications 1 à 3, **caractérisées en ce que** R¹ et/ou R², indépendamment l'un de l'autre, représentent -CF₃, -C₂F₅, -C₃F₇ ou -OCF₃, où n a une valeur de 1 à 6.

7. Utilisation des composés de la formule I selon l'une des revendications 1 à 6 en tant que composants de milieux cristallins liquides.

8. Milieu cristallin liquide ayant deux ou plus de deux composants cristallins liquides, **caractérisé en ce qu'**il comprend au moins un composé de la formule I selon l'une des revendications 1 à 6.

9. Elément d'affichage à cristaux liquides, **caractérisé en ce qu'**il contient un milieu cristallin liquide selon la revendication 8.

10. Elément d'affichage à cristaux liquides, **caractérisé en ce qu'**il contient, en tant que diélectrique, un milieu cristallin liquide selon la revendication 8.

## Revendications (Revendications pour l'(les) Etat(s) contractant(s) suivant(s): DE)

1. 6-Cyclohexyle-trans-décalines substituées-2,4' de la formule I 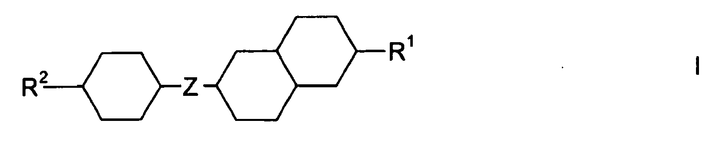 dans laquelle
R¹ représente halogène ou alkyle ayant 1 à 18 atomes de C, dans lequel, en addition, un ou deux groupes CH₂ non adjacents peuvent être remplacés par -O-, -S-, -CO-, -O-CO-, -CO-O- et/ou -C≡C- et dans lequel au moins un atome de H est substitué par un atome d'halogène,
R² représente H, halogène, -CN ou alkyle ayant 1 à 18 atomes de C, dans lequel, en addition, un ou deux groupes CH₂ non adjacents peuvent être remplacés par -O-, -S-, -CO-, -O-CO-, -CO-O- et/ou -C≡C- et/ou dans lequel, en addition, un ou plusieurs atomes de H peuvent être remplacés par halogène, et
Z représente -CH₂O-, -OCH₂-, -COO-, -C≡C-, -CH=CH-, -CF₂O-, -OCF₂-, -CF=CF-, -C₂F₄-, -CH=CH-(CH₂)₂- ou -(CH₂)₄-.

2. 6-Cyclohexyle-trans-décalines substituées-2,4' de la formule I selon la revendication 1, **caractérisées en ce que** R² représente H, méthyle, éthyle, n-propyle, n-butyle, n-pentyle, n-hexyle, n-heptyle ou n-octyle.

3. 6-Cyclohexyle-trans-décalines substituées-2,4' de la formule I selon la revendication 1 ou 2, **caractérisées en ce que** R¹ contient deux ou plus de deux atomes de F.

4. 6-Cyclohexyle-trans-décalines substituées-2,4' de la formule I selon l'une des revendications 1 à 3, **caractérisées en ce que** R¹ est perfluoré.

5. 6-Cyclohexyle-trans-décalines substituées-2,4' de la formule I selon l'une des revendications 1 à 4, **caractérisées en ce que** R¹ et/ou R², indépendamment l'un de l'autre, représentent alkyle, alcoxy ou oxaalkyle ayant 1 à 8 atomes de C, où au moins un atome de H dans R¹ est substitué par un atome d'halogène.

6. 6-Cyclohexyle-trans-décalines substituées-2,4' de la formule I selon l'une des revendications 1 à 3, **caractérisées en ce que** R¹ et/ou R², indépendamment l'un de l'autre, représentent -CF₃, -C₂F₅, -C₃F₇ ou -OCF₃, où n a une valeur de 1 à 6.

7. Utilisation des composés de la formule I selon l'une des revendications 1 à 6 en tant que composants de milieux cristallins liquides.

8. Milieu cristallin liquide ayant deux ou plus de deux composants cristallins liquides, **caractérisé en ce qu'**il comprend au moins un composé de la formule I selon l'une des revendications 1 à 6.

9. Elément d'affichage à cristaux liquides, **caractérisé en ce qu'**il contient un milieu cristallin liquide selon la revendication 8.

10. Elément d'affichage à cristaux liquides, **caractérisé en ce qu'**il contient, en tant que diélectrique, un milieu cristallin liquide selon la revendication 8.
